(19) Europäisches Patentamt
European Patent Office
Office européen des brevets

(11) EP 2 764 005 B1

(12) **FASCICULE DE BREVET EUROPEEN**

(45) Date de publication et mention de la délivrance du brevet:
**12.08.2015 Bulletin 2015/33**

(21) Numéro de dépôt: **12768808.3**

(22) Date de dépôt: **02.10.2012**

(51) Int Cl.:
*C07H 13/04* (2006.01) *C08G 18/00* (2006.01)
*C08L 67/00* (2006.01)

(86) Numéro de dépôt international:
**PCT/EP2012/069482**

(87) Numéro de publication internationale:
**WO 2013/050377 (11.04.2013 Gazette 2013/15)**

(54) **PRÉPARATION DE POLYURÉTHANES ET POLYESTERS À PARTIR DE COMPOSÉS DE TYPE GLYCOLIPIDE**

HERSTELLUNG VON POLYURETHANEN UND POLYESTERN AUS GLYKOLIPIDVERBINDUNGEN

PREPARATION OF POLYURETHANES AND POLYESTERS FROM GLYCOLIPID TYPE COMPOUNDS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.10.2011 FR 1158911**

(43) Date de publication de la demande:
**13.08.2014 Bulletin 2014/33**

(73) Titulaires:
- **Université de Bordeaux**
  **33000 Bordeaux (FR)**
- **Institut des Corps Gras Etudes et Recherches Techniques-Iterg**
  **33600 Pessac (FR)**
- **Centre National de la Recherche Scientifique (CNRS)**
  **75016 Paris (FR)**
- **Institut National des Sciences Appliquées de Lyon**
  **69100 Villeurbanne (FR)**

(72) Inventeurs:
- **CRAMAIL, Henri**
  **F-33350 Sainte Terre (FR)**
- **BOYER, Aurélie**
  **F-33000 Bordeaux (FR)**
- **EPOUNE LINGOME, Cédric**
  **80000 Amiens (FR)**
- **ALFOS, Carine**
  **F-33600 Pessac (FR)**
- **GADENNE, Benoit**
  **F-33110 Le Bouscat (FR)**
- **CLOUTET, Eric**
  **F-33880 Saint Caprais De Bordeaux (FR)**
- **QUENEAU, Yves**
  **F-69004 Lyon (FR)**
- **MOEBS, Sylvie**
  **F-01800 Villieu (FR)**

(74) Mandataire: **Blot, Philippe Robert Emile**
**Cabinet Lavoix**
**2, place d'Estienne d'Orves**
**75441 Paris Cedex 09 (FR)**

(56) Documents cités:
- **SHARMILA MUTHUKRISHNAN ET AL: "Synthesis and Characterization of Methacrylate-Type Hyperbranched Glycopolymers via Self-Condensing Atom Transfer Radical Copolymerization", MACROMOLECULES, vol. 38, no. 8, 1 avril 2005 (2005-04-01), pages 3108-3119, XP055027456, ISSN: 0024-9297, DOI: 10.1021/ma050091q cité dans la demande**
- **JULIEN BERNARD ET AL: "Synthesis of Various Glycopolymer Architectures via RAFT Polymerization: From Block Copolymers to Stars", BIOMACROMOLECULES, vol. 7, no. 1, 1 janvier 2006 (2006-01-01) , pages 232-238, XP055027458, ISSN: 1525-7797, DOI: 10.1021/bm0506086 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 764 005 B1

Printed by Jouve, 75001 PARIS (FR)

(Cont. page suivante)

- RAVIN NARAIN ET AL: "Synthesis and Aqueous Solution Properties of Novel Sugar Methacrylate-Based Homopolymers and Block Copolymers", BIOMACROMOLECULES, vol. 4, no. 6, 1 novembre 2003 (2003-11-01), pages 1746-1758, XP055027459, ISSN: 1525-7797, DOI: 10.1021/bm034166e cité dans la demande

## Description

La présente invention a pour objet de nouveaux polyuréthanes et de nouveaux polyesters. La présente invention a également pour objet de nouveaux monomères ainsi que leur procédé de préparation et leur utilisation pour la synthèse de polyuréthanes et polyesters.

La plupart des glycopolymères (polymères dont la structure comporte des groupements saccharidiques) synthétisés sont issus de dérivés vinyliques (Narain R. et al., Eur. Polym. J., 2002, 38, 273-280), acryliques (Bernard, J. et al., Biomacromolecules, 2005, 7, p. 232-238 ; Barros M.T. et al., Eur. Polym. J., 2009, 45, p. 295-301 ; Narain R. et al., Biomacromolecules, 2003, 4, p. 1746-1758) ou acryloyles (Muthukrishnan, H. et al., Macromolecules, 2005, 38, p. 3108-3119) sur lesquels sont incorporés des groupements saccharides pendants. La synthèse de polyuréthanes linéaires issus de dérivés glycosidiques a été très peu étudiée car il est difficile d'obtenir un monomère possédant une fonctionnalité de deux, la plupart des sucres possédant plus de 4 fonctions alcool réactives. Par ailleurs, plusieurs travaux ont porté sur la synthèse de polyuréthanes issus de corps gras. Ces derniers présentent des propriétés thermomécaniques limitées, telles qu'une température de transition vitreuse basse et des modules faibles.

Il existe donc un besoin de disposer de polyuréthanes présentant des propriétés thermo-mécaniques améliorées par rapport aux polyuréthanes issus de dérivés de corps gras.

La présente invention a pour but de fournir des polyuréthanes et polyesters possédant de bonnes propriétés thermo-mécaniques, à partir de dérivés de type glycolipide.

La présente invention a également pour but de fournir de nouveaux monomères de type glycolipide ainsi que leur utilisation pour la préparation de polyuréthanes et polyesters.

Un des buts de la présente invention est de fournir un procédé de préparation de polyuréthanes et de polyesters, sous forme linéaire.

Un autre but de la présente invention est de fournir un procédé de préparation de polyuréthanes et polyesters, sous forme de réseau.

Un autre but de l'invention est d'utiliser les polyuréthanes et polyesters obtenus selon l'invention pour des applications à haute valeur ajoutée, notamment dans le domaine médical et pharmaceutique.

Selon un premier aspect, la présente invention concerne l'utilisation d'un composé de formule (I) :

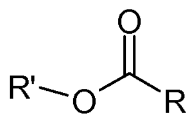
(I)

dans laquelle :

- R représente un groupe alkyle linéaire ou ramifié, comprenant de 3 à 27 atomes de carbone, de préférence de 8 à 27 atomes de carbone, ledit groupe alkyle étant substitué par au moins deux groupes hydroxyle, et pouvant éventuellement contenir une ou plusieurs insaturations ;et
- R' est choisi parmi les sucres ou les sucre-alcools ;
  pour la préparation de polymères choisis parmi les polyuréthanes et les polyesters.

De préférence, la présente invention concerne l'utilisation d'un composé de formule (I) telle que définie ci-dessus pour la préparation de polyuréthanes.

Dans le cadre de cette invention, et sauf mention contraire, on entend par « sucre », un glucide choisi parmi les oses et les osides, et comprenant de 1 à 10 unités monosaccharidiques. Par exemple, en guise de sucre selon l'invention, on trouve le thréose, l'érythrose, le désoxyribose, le ribose, le xylose, le ribulose, le lyxose, le glucose, le méthyl glucoside, le mannose, le fructose, l'idose, le sorbose, le galactose, l'allose, le maltose, le lactose, l'isomaltose, l'isomaltulose, le cellobiose, et le saccharose, le raffinose, le mélézitose. Les osides comportent une chaine glycosidique alkyle linéaire ou ramifiée comprenant de 1 à 12 atomes de carbone. De préférence, le sucre selon l'invention est choisi parmi le méthyl glucoside et le saccharose.

Dans le cadre de cette invention, et sauf mention contraire, on entend par « sucre-alcool », un dérivé de sucre tel que défini selon l'invention, et comprenant de 1 à 12 fonctions hydroxyles. Un « sucre-alcool » peut notamment être un sucre sur lequel est greffée une chaîne comprenant une à plusieurs fonctions hydroxyles ou un sucre dont la fonction réductrice a été hydrogénée. Selon l'invention, le terme « sucre-alcool » peut être assimilé au terme « dérivés de sucre ». Par exemple, à titre de sucre-alcool selon l'invention, on trouve le sorbitol, l'isomalt, le xylitol, le mannitol et l'arabinitol. De préférence, les sucre-alcools selon l'invention sont choisis parmi le sorbitol et l'isomalt.

Selon un autre aspect, l'invention concerne des composés de formule (I) :

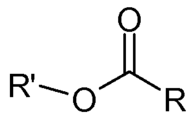 (I)

dans laquelle :

- R représente un groupe alkyle linéaire ou ramifié, comprenant de 3 à 27 atomes de carbone, de préférence de 8 à 27 atomes de carbone, ledit groupe alkyle étant substitué par au moins deux groupes hydroxyles, et pouvant éventuellement contenir une ou plusieurs insaturations ; et
- R' est choisi parmi les sucres ou les sucre-alcools.

Selon un mode de réalisation, R représente un groupe alkyle linéaire ou ramifié, comprenant de 10 à 25 atomes de carbone, de préférence de 15 à 20 atomes de carbone. En particulier, R représente un groupe alkyle linéaire ou ramifié, comprenant de 17 atomes de carbone.

Selon un mode de réalisation, R représente un groupe alkyle linéaire.

Selon un mode de réalisation, R est substitué par au moins deux groupes hydroxyles, de préférence par 2 à 4 groupes hydroxyles. De préférence, R est substitué par deux groupes hydroxyles.

Selon un mode de réalisation, R comprend une insaturation.

Selon un mode de réalisation, l'invention concerne des composés de formule (I) :

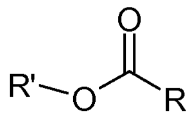 (I)

dans laquelle :

- R représente un groupe alkyle linéaire ou ramifié, comprenant de 8 à 27 atomes de carbone, ledit groupe alkyle étant substitué par au moins deux groupes hydroxyles, et pouvant éventuellement contenir une insaturation ; et
- R' est choisi parmi les sucres ou les sucre-alcools.

Selon un mode de réalisation, la présente invention concerne un composé de formule (I) tel que défini précédemment, dans lequel R répond à la formule (A) :

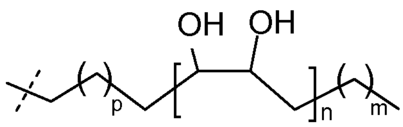

(A)

dans laquelle :

- n est un entier compris de 1 à 3 ;
- m est un entier compris de 1 à 6 ;
- p est un entier compris de 1 à 9.

Dans le cadre de l'invention, et sauf mention contraire, la liaison sur laquelle se trouve le sigle 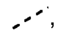, signifie que ladite liaison est reliée au carbone de la fonction carbonyle du composé (I). Ainsi, dans le cadre de l'invention, le composé de formule (I) peut s'écrire selon les deux façons suivantes :

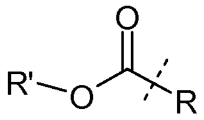 ou 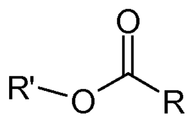 (I)

Selon un mode de réalisation particulier, dans la formule (I) susmentionnée, R répond à la formule (A-1) suivante :

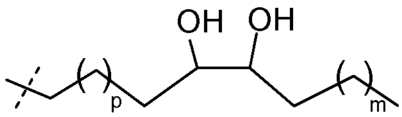
(A-1)

dans laquelle :

- m est un entier compris de 1 à 6 ; et
- p est un entier compris de 1 à 9.

Les composés de formule (I) pour lesquels R répond à la formule (A-1) correspondent à des composés de formule (I) où R répond à la formule (A) et dans laquelle n vaut 1.

De préférence, dans la formule (I) susmentionnée, R répond à la formule (A-2) suivante :

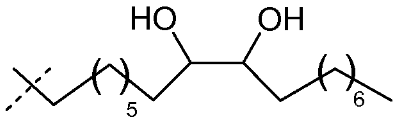
(A-2)

Les composés de formule (I) pour lesquels R répond à la formule (A-2) correspondent à des composés de formule (I) où R répond à la formule (A) et dans laquelle n vaut 1, p vaut 5 et m est égale à 6.

Selon un mode de réalisation, la présente invention concerne un composé de formule (I) tel que défini précédemment, dans lequel R' est choisi dans le groupe constitué du thréose, l'érythrose, le désoxyribose, le ribose, le xylose, le ribulose, le lyxose, le glucose, le méthyl glucoside, le mannose, le fructose, l'idose, le sorbose, le galactose, l'allose, le maltose, le lactose, l'isomaltose, l'isomaltulose, le cellobiose, le saccharose, le raffinose, le mélézitose, le sorbitol, l'isomalt, le xylitol, le mannitol et l'arabinitol.

Selon un mode de réalisation, R' est choisi dans le groupe constitué du thréose, de l'érythrose, du désoxyribose, du ribose, du xylose, du ribulose, du lyxose, du méthyl glucoside, de l'idose, du galactose, de l'allose, du maltose, du lactose, de l'isomaltose, de l'isomaltulose, du cellobiose, du saccharose, du raffinose, du mélézitose, du sorbitol, de l'isomalt, du xylitol et de l'arabinitol.

Préférentiellement, R' est choisi dans le groupe constitué du sorbitol, de l'isomalt, du méthyl glucoside et du saccharose.

Selon un mode de réalisation particulier, la présente invention concerne des composés de formule (I), caractérisés en ce que R' répond à la formule (B) suivante :

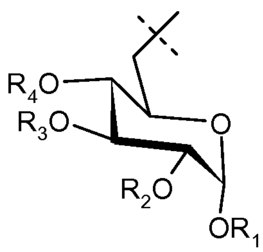
(B)

dans laquelle $R_1$, $R_2$, $R_3$ et $R_4$ représentent, indépendamment les uns des autres : H ; un groupe alkyle comprenant de 1 à 12 atomes de carbone; un groupe $CH_3C(O)$- ; un groupe arylalkyle comprenant de 6 à 12 atomes de carbone; un

groupe ((C1-C6)alkyle)$_3$-Si- ou $R_1$ et $R_2$, $R_2$ et $R_3$, ou $R_3$ et $R_4$, forment ensemble un groupe isopropylidène.

De préférence, $R_1$, $R_2$, $R_3$ et $R_4$ sont indépendamment les uns des autres choisis parmi H et un groupe alkyle comprenant de 1 à 12 atomes de carbone.

Selon l'invention, des composés préférés sont ceux pour lesquels $R_1$ représente méthyle, et $R_2$, $R_3$ et $R_4$ représentent H.

La présente invention peut également concerner des composés de formule (I) dans laquelle R' peut répondre à la formule (B') :

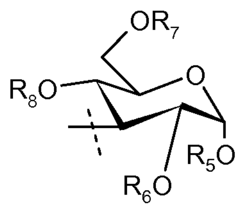
(B')

dans laquelle $R_5$, $R_6$, $R_7$ et $R_8$ représentent, indépendamment les uns des autres : H ; un groupe alkyle comprenant de 1 à 12 atomes de carbone; un groupe $CH_3C(O)$- ; un groupe arylalkyle comprenant de 6 à 12 atomes de carbones; un groupe ((C1-C6)alkyle)$_3$-Si- ou $R_5$ et $R_6$ ou $R_7$ et $R_8$, forment ensemble un groupe isopropylidène.

De préférence, $R_5$, $R_6$, $R_7$ et $R_8$ sont indépendamment les uns des autres choisis parmi H et un groupe alkyle comprenant de 1 à 12 atomes de carbone.

Selon l'invention, des composés préférés sont ceux pour lesquels $R_5$ représente méthyle, et $R_6$, $R_7$ et $R_8$ représentent H.

Selon un autre mode de réalisation, la présente invention concerne des composés de formule (I) pour laquelle R' répond à la formule (C) :

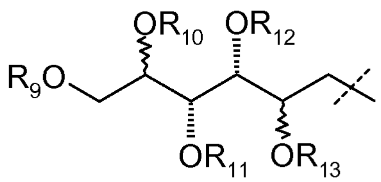
(C)

dans laquelle $R_9$, $R_{10}$, $R_{11}$, $R_{12}$ et $R_{13}$ représentent, indépendamment les uns des autres : H ; un groupe alkyle comprenant de 1 à 12 atomes de carbone ; un groupe $CH_3C(O)$- ; un groupe arylalkyle comprenant de 6 à 12 atomes de carbones; un groupe ((C1-C6)alkyle)$_3$-Si- ou $R_9$ et $R_{10}$, ou $R_{10}$ et $R_{11}$, ou $R_{11}$ et $R_{12}$, ou $R_{12}$ et $R_{13}$, forment ensemble un groupe isopropylidène.

De préférence, $R_9$, $R_{10}$, $R_{11}$, $R_{12}$ et $R_{13}$ sont indépendamment les uns des autres choisis parmi H et un groupe alkyle comprenant de 1 à 12 atomes de carbone.

Des composés préférés de formule (I) susmentionnée, sont ceux pour lesquels R' répond à l'une quelconque des formules suivantes :

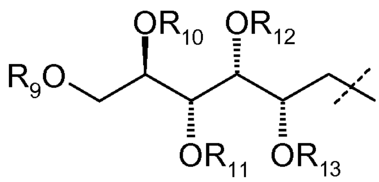
(C-1)

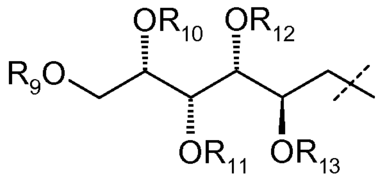

(C-2)

Selon l'invention, des composés préférés sont ceux pour lesquels $R_9$, $R_{10}$, $R_{11}$, $R_{12}$ et $R_{13}$ représentent H.

Dans le cadre de l'invention, et sauf mention contraire, 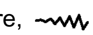 représente une liaison qui peut être en avant ou en arrière du plan que forme la chaîne aliphatique principale.

Selon un autre mode de réalisation, la présente invention concerne des composés de formule (I) pour laquelle R' répond à la formule (D) suivante:

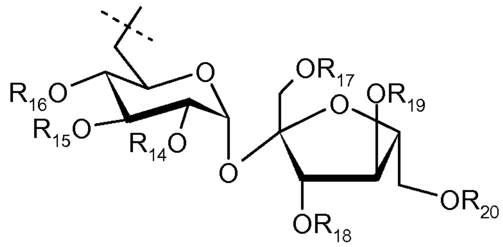

(D)

dans laquelle $R_{14}$, $R_{15}$, $R_{16}$, $R_{17}$, $R_{18}$, $R_{19}$ et $R_{20}$ représentent, indépendamment les uns des autres : H ; un groupe alkyle comprenant de 1 à 12 atomes de carbone ; un groupe $CH_3C(O)$- ; un groupe arylalkyle comprenant de 6 à 12 atomes de carbones ; un groupe ((C1-C6)alkyle)$_3$-Si- ou $R_{14}$ et $R_{17}$ ou $R_{19}$ et $R_{20}$ forment ensemble un groupe isopropylidène.

De préférence, $R_{14}$, $R_{15}$, $R_{16}$, $R_{17}$, $R_{18}$, $R_{19}$ et $R_{20}$ sont indépendamment les uns des autres choisis parmi H et un groupe alkyle comprenant de 1 à 12 atomes de carbone.

Selon l'invention, des composés préférés sont ceux pour lesquels $R_{14}$, $R_{15}$, $R_{16}$, $R_{17}$, $R_{18}$, $R_{19}$ et $R_{20}$ représentent H.

Selon un autre mode de réalisation, la présente invention concerne des composés de formule (I) pour laquelle R' répond à la formule (D') suivante:

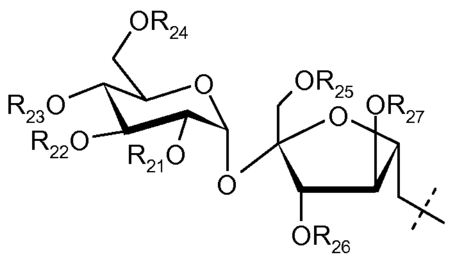

(D')

dans laquelle $R_{21}$, $R_{22}$, $R_{23}$, $R_{24}$, $R_{25}$, $R_{26}$ et $R_{27}$ représentent, indépendamment les uns des autres: H ; un groupe alkyle comprenant de 1 à 12 atomes de carbone ; un groupe $CH_3C(O)$- ; un groupe arylalkyle comprenant de 6 à 12 atomes de carbones ; un groupe ((C1-C6)alkyle)$_3$-Si- ou $R_{23}$ et $R_{24}$ ou $R_{21}$ et $R_{25}$ ou $R_{26}$ et $R_{27}$ forment ensemble un groupe isopropylidène.

De préférence, $R_{21}$, $R_{22}$, $R_{23}$, $R_{24}$, $R_{25}$, $R_{26}$ et $R_{27}$ sont indépendamment les uns des autres choisis parmi H et un groupe alkyle comprenant de 1 à 12 atomes de carbone.

Selon l'invention, des composés préférés sont ceux pour lesquels $R_{21}$, $R_{22}$, $R_{23}$, $R_{24}$, $R_{25}$, $R_{26}$ et $R_{27}$ représentent H.

Selon un autre mode de réalisation, la présente invention concerne des composés de formule (I) pour laquelle R' répond à la formule (E) suivante:

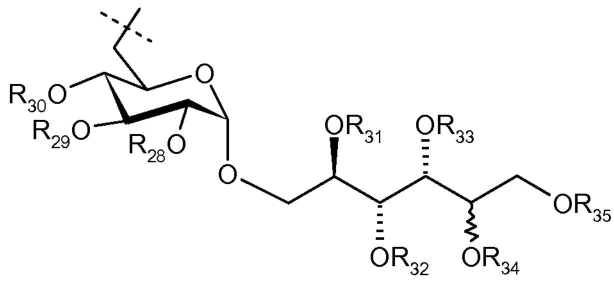

(E)

dans laquelle $R_{28}$, $R_{29}$, $R_{30}$, $R_{31}$, $R_{32}$, $R_{33}$, $R_{34}$ et $R_{35}$ représentent, indépendamment les uns des autres : H ; un groupe alkyle comprenant de 1 à 12 atomes de carbone ; un groupe $CH_3C(O)$- ; un groupe arylalkyle comprenant de 6 à 12 atomes de carbones ; un groupe ((C1-C6)alkyle)$_3$-Si- ou $R_{29}$ et $R_{30}$ ou $R_{31}$ et $R_{32}$ ou $R_{32}$ et $R_{33}$ ou $R_{33}$ et $R_{34}$ forment ensemble un groupe isopropylidène.

De préférence, $R_{28}$, $R_{29}$, $R_{30}$, $R_{31}$, $R_{32}$, $R_{33}$, $R_{34}$ et $R_{35}$ sont indépendamment les uns des autres choisis parmi H et un groupe alkyle comprenant de 1 à 12 atomes de carbone. Selon l'invention, des composés préférés sont ceux pour lesquels $R_{28}$, $R_{29}$, $R_{30}$, $R_{31}$, $R_{32}$, $R_{33}$, $R_{34}$ et $R_{35}$ représentent H.

Selon un autre mode de réalisation, la présente invention concerne des composés de formule (I) pour laquelle R' répond à la formule (E') suivante:

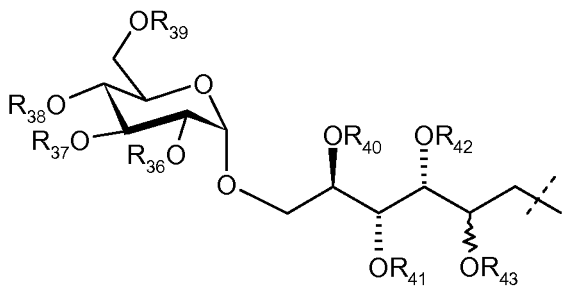

(E')

dans laquelle $R_{36}$, $R_{37}$, $R_{38}$, $R_{39}$, $R_{40}$, $R_{41}$, $R_{42}$ et $R_{43}$ représentent, indépendamment les uns des autres : H ; un groupe alkyle comprenant de 1 à 12 atomes de carbone ; un groupe $CH_3C(O)$- ; un groupe arylalkyle comprenant de 6 à 12 atomes de carbones ; un groupe ((C1-C6)alkyle)$_3$-Si- ou $R_{37}$ et $R_{38}$ ou $R_{38}$ et $R_{39}$ ou $R_4$o et $R_{41}$ ou $R_{41}$ et $R_{42}$, ou $R_{42}$ et $R_{43}$ forment ensemble un groupe isopropylidène.

De préférence, $R_{36}$, $R_{37}$, $R_{38}$, $R_{39}$, $R_{40}$, $R_{41}$, $R_{42}$ et $R_{43}$ sont indépendamment les uns des autres choisis parmi H et un groupe alkyle comprenant de 1 à 12 atomes de carbone.

Des composés préférés de formule (I) susmentionnée, sont ceux pour lesquels R' répond à la formule suivante :

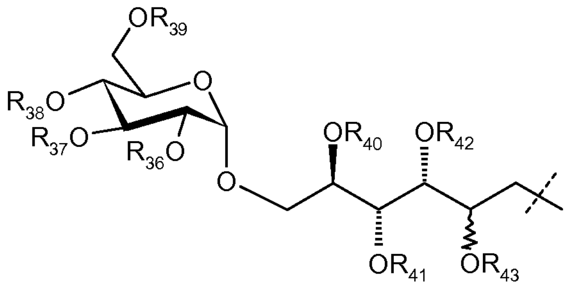

(E'-1)

Selon l'invention, des composés préférés sont ceux pour lesquels $R_{36}$, $R_{37}$, $R_{38}$, $R_{39}$, $R_{40}$, $R_{41}$, $R_{42}$ et $R_{43}$ représentent H.

Selon l'invention, les groupes $R_1$ à $R_{43}$ peuvent représenter tout groupe protecteur connu des fonctions hydroxyles des sucres, notamment un groupement -$SO_2$-(C6-C10)aryle ou un groupement -$SO_2$-(C1-C6)alkyle.

Les inventeurs ont avantageusement préparé de nouveaux monomères glycosylés dérivés de ressources renouvelables.

Dans le cadre de l'invention, et sauf mention contraire, le terme « monomère glycosylé » ou « synthon

glycosylé » représente un monomère polyol biosourcé issu de la condensation d'une chaîne grasse hydroxylée avec un dérivé contenant un ou plusieurs motifs sucres. Parmi les chaînes grasses, on peut citer les chaînes issues de l'acide oléique, l'acide palmitoléique, l'acide linoléique, l'acide linolénique, l'acide erucique ou l'acide ricinoléique.

Les monomères glycosylés selon l'invention sont également appelés « dérivés glycolipidiques » ou « dérivés glycolipides », car ils dérivent à la fois de chaîne grasse et de sucres.

Les composés de formule (I) susmentionnée comportent donc à la fois une partie « chaîne grasse » et une partie « sucre » au sein de leur structure, qui comprennent chacune de une à plusieurs fonctions alcools.

Les structures de ces polyols biosourcés selon l'invention s'avèrent très intéressantes en raison des multiples fonctions alcools présentes sur lesdits polyols, qui en font des précurseurs potentiels de réseaux de polymères, et notamment de réseaux de polyuréthanes. Ces monomères sont avantageusement des précurseurs fonctionnels à fonctionnalité contrôlée.

Par ailleurs, la pureté des monomères est importante car elle peut permettre d'optimiser les propriétés des polymères obtenus selon l'invention.

Les composés de formule (I) susmentionnée peuvent être utilisés à titre de tensioactifs.

Parmi les radicaux "alkyle", on peut notamment citer, lorsqu'ils sont linéaires, les radicaux méthyle, éthyle, propyle, butyle, pentyle, hexyle, octyle, nonyle et décyle. On peut notamment citer, lorsqu'ils sont ramifiés ou substitués par un ou plusieurs radicaux alkyles, les radicaux isopropyle, tert-butyle, 2-éthylhexyle, 2-méthylbutyle, 2-méthylpentyle, 1-méthylpentyle et 3-méthylheptyle.

Selon la présente invention, les radicaux "alkylène" représentent des radicaux (également nommés alkylidènes) dérivés des alcanes dont les deux atomes d'hydrogène terminaux ont été supprimés. Lorsque lesdits radicaux alkylènes sont linéaires, ils peuvent être représentés par la formule $-(CH_2)_k-$, k correspondant au nombre d'atomes de carbone de l'alcane dont est issu le radical alkylène.

Selon la présente invention, les radicaux « aryles » représentent des mono- ou bi-cycles hydrocarbonés comprenant de 6 à 14 atomes de carbones, éventuellement substitué. On peut notamment citer le phényle ou l'anthracène.

Selon la présente invention, on entend par radicaux « cycloalkylène », des radicaux dérivés des cycloalkanes dont un atome d'hydrogène terminal a été supprimé.

Selon l'invention, les radicaux cycloalkylènes peuvent être substitués par un ou plusieurs groupes (C1-C6)alkyles.

Dans le cadre de l'invention, et sauf mention contraire, on entend par (C1-C6)alkyle, un groupe alkyle comprenant de 1 à 6 atomes de carbone.

Dans le cadre de l'invention, et sauf mention contraire, on entend par (C6-C10)aryle, un groupe aryle comprenant de 6 à 10 atomes de carbone.

L'expression "arylène" désigne un radical (également nommé arènediyle) dérivé des arènes dont deux atomes d'hydrogène du cycle ont été supprimés. Parmi les radicaux arylènes, on peut par exemple citer les radicaux o-phénylène ou benzène-1,2-diyle. Selon la présente invention, les radicaux « arylalkyles » représentent un radical alkyle substitué par un groupe aryle. Les radicaux arylalkyle sont des radicaux aryl-alkyl-, le groupes aryles et alkyles étant tels que définis ci-dessus. Parmi les radicaux arylalkyles, on peut notamment citer le radical benzyle ou phénéthyle. Ces groupes arylalkyles peuvent être substitués par ou un plusieurs substituants choisis parmi amino, hydroxy, halogène, alkyle ou alcoxy.

Selon l'invention, le radical « cycloalkyle » représente tout groupement non aromatique mono ou bi-cyclique, contenant de 4 à 10 atomes de carbones. On peut notamment citer le cyclobutyle, le cyclopentyle, le cyclohexyle, le cycloheptyle, le cyclooctyle.

Selon un autre aspect, la présente invention concerne un polymère susceptible d'être obtenu par polymérisation d'un composé de formule (I) tel que défini précédemment et d'un (poly)isocyanate. Le polymère obtenu selon ce mode de réalisation est un homopolymère, de type polyuréthane.

Selon un autre aspect, la présente invention concerne un polyester susceptible d'être obtenu par réaction d'un composé de formule (I) tel que défini précédemment avec par exemple un di-chlorure d'acide.

Selon un mode de réalisation, le (poly)isocyanate utilisé selon l'invention peut être un diisocyanate répondant à la formule (O)CN-$A_3$-NC(O), dans laquelle $A_3$ représente :

- un groupe alkylène, linéaire ou ramifié, comprenant de 2 à 20 atomes de carbone ; ou
- un groupe cycloalkylène-alkylène-cycloalkylène, comprenant de 6 à 30 atomes de carbone ; ou
- un groupe arylène-alkylène-arylène, comprenant de 6 à 30 atomes de carbone ; ou
- un groupe cycloalkylène, comprenant de 3 à 10 atomes de carbone ;
- un groupe alkylène-cycloalkylène, comprenant de 3 à 15 atomes de carbone ; ou
- un groupe arylène comprenant de 6 à 10 atomes de carbone.

Préférentiellement, le diisocyanate est choisi parmi le groupe constitué du: 1,6-hexaméthylène diisocyanate

(HDI), 1,12-dodécane diisocyanate, cyclobutane 1,3-diisocyanate, cyclohexane 1,3 et/ou 1,4-diisocyanate, 1-isocyanato 3,3,5-triméthyl-5-diisocyanato-méthyl cyclohexane (IPDI), 1, 3- et/ou 1,4-phénylène diisocyanate, 2,4- et/ou 2, 6-toluylène diisocyanate, diphénylméthane 4,4'-diisocyanate (MDI), 1, 3-bisisocyanatométhyl cyclohexane et methylène-bis(4-cyclohexylisocyanate).

De préférence, le diisocyanate est le 1-isocyanato 3,3,5-triméthyl-5-diisocyanato-méthyl cyclohexane (IPDI).

Selon un mode de réalisation particulier, le polymère susceptible d'être obtenu par polymérisation d'un composé (I) et d'un (poly)isocyanate tels que définis précédemment, répond à la formule (IV) suivante :

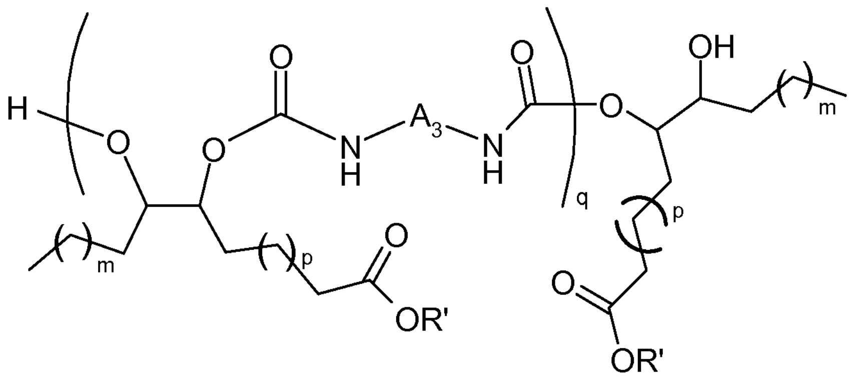

(IV)

dans laquelle :

- n est 1 ;
- m est compris de 1 à 6 ;
- p est compris de 1 à 9 ;
- R' est choisi parmi les sucres ou les sucre-alcools ;
- $A_3$ est choisi parmi le groupe constitué de :
  - un groupe alkylène, linéaire ou ramifié, comprenant de 2 à 20 atomes de carbone ;
  - un groupe cycloalkylène-alkylène-cycloalkylène, comprenant de 6 à 30 atomes de carbone ;
  - un groupe arylène-alkylène-arylène, comprenant de 6 à 30 atomes de carbone ;
  - un groupe cycloalkylène, comprenant de 3 à 10 atomes de carbone ;
  - un groupe alkylène-cycloalkylène, comprenant de 3 à 15 atomes de carbone ; et
  - un groupe arylène comprenant de 6 à 10 atomes de carbone ;
- q représente un nombre entier compris entre 2 à 500000, de préférence de 2 à 50000, notamment de 2 à 5000, de préférence de 2 à 500 et préférentiellement de 2 à 50

Préférentiellement, le polymère susceptible d'être obtenu par polymérisation d'un composé (I) et de l'IPDI, à titre de (poly)isocyanate tels que définis précédemment, répond à la formule (IV-1) suivante :

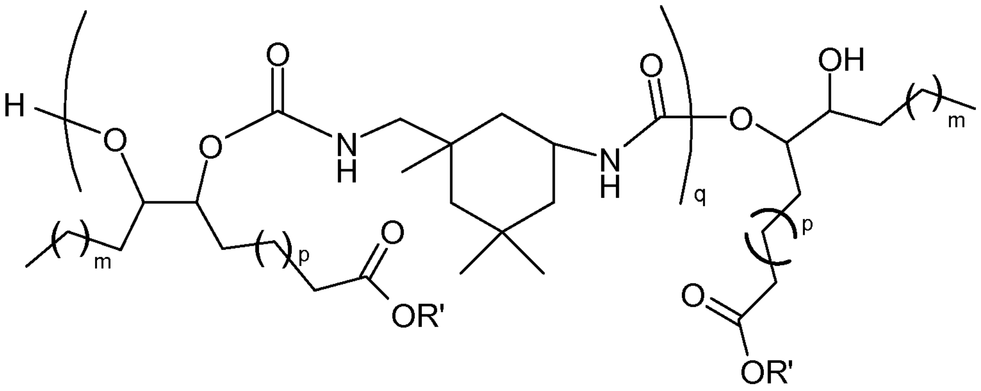

(IV-1)

En particulier, dans la formule (IV-1) susmentionnée, m vaut 6 et p vaut 5.

Des polymères préférés selon l'invention, sont ceux pour lesquels dans les formules (IV) ou (IV-1) susmentionnées, R' répond à la formule (B) telle que définie précédemment. De préférence, dans la formule (B), $R_1$ représente méthyle et $R_2$, $R_3$ et $R_4$ représentent H. Des polymères préférés selon l'invention, sont ceux pour lesquels dans les formules (IV) ou (IV-1) susmentionnées, R' répond à la formule (B') telle que définie précédemment. De préférence, dans la formule (B'), $R_5$ représente méthyle et $R_6$, $R_7$ et $R_8$ représentent H. Des polymères préférés selon l'invention, sont ceux pour lesquels dans les formules (IV) ou (IV-1) susmentionnées, R' répond à la formule (C) telle que définie précédemment. De préférence, dans la formule (C), $R_9$, $R_{10}$, $R_{11}$, $R_{12}$ et $R_{13}$ représentent H.

Des polymères préférés selon l'invention, sont ceux pour lesquels dans les formules (IV) ou (IV-1) susmentionnées, R' répond à la formule (D) telle que définie précédemment. De préférence, dans la formule (D), $R_{14}$, $R_{15}$, $R_{16}$, $R_{17}$, $R_{18}$, $R_{19}$ et $R_{20}$ représentent H.

Des polymères préférés selon l'invention, sont ceux pour lesquels dans les formules (IV) ou (IV-1) susmentionnées, R' répond à la formule (D') telle que définie précédemment. De préférence, dans la formule (D'), $R_{20}$, $R_{21}$, $R_{22}$, $R_{23}$, $R_{24}$, $R_{25}$, $R_{26}$ et $R_{27}$ représentent H. Des polymères préférés selon l'invention, sont ceux pour lesquels dans les formules (IV) ou (IV-1) susmentionnées, R' répond à la formule (E) telle que définie précédemment. De préférence, dans la formule (E), $R_{28}$, $R_{29}$, $R_{30}$, $R_{31}$, $R_{32}$, $R_{33}$, $R_{34}$ et $R_{35}$ représentent H. Des polymères préférés selon l'invention, sont ceux pour lesquels dans les formules (IV) ou (IV-1) susmentionnées, R' répond à la formule (E') telle que définie précédemment. De préférence, dans la formule (E'), $R_{36}$, $R_{37}$, $R_{38}$, $R_{39}$, $R_{40}$, $R_{41}$, $R_{42}$ et $R_{43}$ représentent H. Selon un autre aspect, la présente invention concerne un polymère susceptible d'être obtenu par polymérisation d'un composé (I) tel que défini précédemment, d'un (poly)isocyanate et d'un diol.

Selon un mode de réalisation, la présente invention concerne un polymère susceptible d'être obtenu par polymérisation d'un composé (I) tel que défini précédemment, d'un (poly)isocyanate et d'un diol choisi parmi les alcanediols, les polyalkylène-diols, les polyether-diols, les polyester-diols et des diols présentant l'une des formules suivantes :

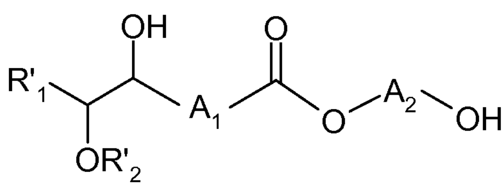

(II)

dans laquelle :

- $R'_1$ représente un groupe alkyle, linéaire ou ramifié, comprenant de 2 à 14 atomes de carbone ;
- $R'_2$ représente un groupe alkyle, linéaire ou ramifié, comprenant de 1 à 8 atomes de carbone ;
- $A_1$ représente un radical alkylène divalent, linéaire ou ramifié, comprenant de 2 à 14 atomes de carbone ;
- $A_2$ représente un radical alkylène divalent, linéaire ou ramifié, comprenant de 1 à 10 atomes de carbone ; et

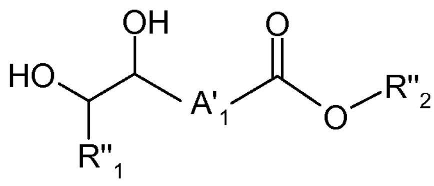

(III)

dans laquelle :

- $R''_1$ représente un groupe alkyle, linéaire ou ramifié, comprenant de 2 à 14 atomes de carbone ;
- $R''_2$ représente un groupe alkyle, linéaire ou ramifié, comprenant de 1 à 8 atomes de carbone ;
- $A'_1$ représente un radical alkylène divalent, linéaire ou ramifié, comprenant de 2 à 14 atomes de carbone.

Les polymères obtenus selon ce mode de réalisation est un copolymère, de type polyuréthane.

Parmi les alcane-diols, on peut citer par exemple, l'éthane-diol, le propane-diol, le butane-diol, le pentane-diol, l'hexane-diol, l'heptane-diol, l'octane-diol, le décane-diol ou le dodécane-diol.

Parmi les polyalkylène-diols, on peut citer par exemple, le polypropylène-glycol, le polyéthylène-glycol.

En particulier, la présente invention concerne un polymère susceptible d'être obtenu par polymérisation d'un composé (I) tel que défini précédemment, d'un (poly)isocyanate et d'un diol de formule (III).

De préférence, dans le diol de formule (III) susmentionnée, $R''_1$ représente un groupe octyle, $R''_2$ représente un groupe éthyle et $A'_1$ représente un radical heptylène.

En particulier, la présente invention concerne un polymère susceptible d'être obtenu par polymérisation d'un composé (I) tel que défini précédemment, d'un (poly)isocyanate et d'un diol de formule (II).

De préférence, dans le diol de formule (II) susmentionnée, $R'_1$ représente un groupe octyle, $R'_2$ représente un groupe éthyle, $A_1$ représente un radical heptylène et $A'_2$ représente un radical pentylène.

Selon un mode de réalisation, le (poly)isocyanate permettant la préparation du polymère selon l'invention est un diisocyanate répondant à la formule (O)CN-$A_3$-NC(O), dans laquelle $A_3$ est tel que défini précédemment.

Selon un autre aspect, l'invention concerne un procédé de préparation de polyuréthane, comprenant une étape de réaction d'un composé de formule (I) tel que défini ci-dessus, avec un diisocyanate de formule (O)CN-$A_3$-NC(O) tel que défini précédemment, à une température comprise de 40°C à 100°C, de préférence à 60°C, dans un solvant. Typiquement, la réaction peut être réalisée en présence d'un catalyseur à une quantité comprise de 0,01 à 0,9% en masse par rapport à la masse totale de réactifs. De préférence, le catalyseur utilisé est le di-butyl dilaurate d'étain à une quantité comprise de 0,02 à 0,1% en masse.

La formation des polyuréthanes selon l'invention peut être confirmée par FTIR (spectroscopie infrarouge) grâce à la disparition de la bande de vibration des fonctions isocyanate à 2250 $cm^{-1}$ ainsi que l'apparition de celle des fonctions uréthane localisée à 1530 $cm^{-1}$. La réaction peut être considérée comme finie lorsque la bande de vibration des fonctions isocyanate n'évolue plus.

Selon un autre aspect, la présente invention concerne un copolymère susceptible d'être obtenu par polymérisation d'au moins deux composés différents de formule (I) et d'un (poly)isocyanate tels que définis précédemment. En particulier, le copolymère est obtenu à partir de deux composés différents de formule (I) et d'un (poly)isocyanate tel que défini précédemment.

Selon la nature du solvant utilisé dans le procédé de l'invention, différents types de polymères peuvent être obtenus. En effet, le procédé selon l'invention peut conduire à un polymère linéaire ou un polymère réseau, selon la solvatation des composés de formule (I) définis précédemment dans le solvant de réaction. Il y a donc une sélectivité de la fonctionnalité du composé de formule (I) selon la nature du solvant de polymérisation. Dans la cadre de l'invention, et sauf mention contraire, on entend par polymère réseau, un polymère réticulé.

Selon un mode de réalisation, le solvant peut être choisi parmi les solvants permettant la solvatation du composé de formule (I) susmentionnée, notamment le DMF, la N-méthylpyrrolidone (NMP) ou le DMSO. De préférence, le solvant utilisé est le DMF. L'emploi de tels solvants peut permettre la solubilisation du composé de formule (I) dans ledit solvant de réaction, ce qui implique que toutes les fonctions alcools du monomère glycolipide portées à la fois par la chaîne grasse et par le sucre peuvent réagir. Ainsi, le procédé réalisé dans un tel solvant peut conduire à la préparation d'un polymère réseau, et plus particulièrement d'un polyuréthane réseau.

Selon un autre mode de réalisation, le solvant est choisi parmi les solvants ne permettant pas la solvatation du composé de formule (I) susmentionnée, notamment le THF, le diéthyléther ou l'acétate d'éthyle. De préférence, le solvant utilisé est le THF. L'emploi de tels solvants ne permet pas la solubilisation du composé de formule (I) dans ledit solvant de réaction, ce qui implique que toutes les fonctions alcools du monomère glycolipide ne peuvent pas réagir. Notamment, la réaction réalisée dans un solvant tel que THF ne permet pas la solubilisation des fonctions alcools portées

par le motif sucre du monomère glycolipide. Dans un tel solvant, lesdits composés peuvent se présenter sous la forme de micelles inverses, dans lesquelles les parties sucres se rassemblent et se retrouvent à l'intérieur desdites micelles, tandis que les parties chaînes grasses des composés (I) se retrouvent à l'extérieur. Ainsi, seules les fonctions alcools portées par la chaîne grasse peuvent réagir. En effet, la partie chaîne grasse est soluble dans un solvant tel que le THF. Bien que la partie chaîne grasse du composé (I) soit soluble, la partie sucre du composé (I) s'avère en revanche insoluble. De ce fait, le solvant de réaction ne permet pas de manière globale la solvatation du composé (I).

Ainsi, le procédé de l'invention réalisé dans un tel solvant peut conduire à la préparation d'un polymère linéaire, et plus particulièrement d'un polyuréthane linéaire, dans lesquels les motifs sucres sont pendants.

Selon l'invention, le ratio entre les fonctions isocyanates et les fonctions alcools du monomère glycolipide utilisé dans le procédé de l'invention, peut également jouer un rôle sur la nature du polymère obtenu selon le procédé. En effet, il est possible d'obtenir une vaste gamme de polyuréthanes allant des oligomères de faibles masses moléculaires linéaires à des polymères réseaux, mais également il est possible d'obtenir des polymères ramifiés. Ainsi il existe une vaste gamme de polymères ramifiés possible, présentant un taux de ramification plus ou moins élevé, selon le ratio entre les fonctions isocyanates et les fonctions alcools du monomère glycolipide utilisé au cours de la réaction de polymérisation. Typiquement, dans un solvant ne permettant pas la solvatation du composé (I) susmentionné, tel que le THF, plus ledit ratio augmente, à savoir plus on introduit de composé (poly)isocyanate, plus le nombre de fonctions hydroxyles de la partie chaîne grasse du monomère glycolipide pouvant réagir augmente, conduisant ainsi à un polymère linéaire. Une fois que toutes les fonctions hydroxyles de la partie chaîne grasse ont réagi, plus le ratio augmente et plus certaines fonctions hydroxyles de la partie sucre vont pouvoir réagir. Il en résulte ainsi la formation de ramifications et donc l'obtention de polymère ramifié. De ce fait, plus le ratio entre les fonctions isocyanates et les fonctions hydroxyles augmente et plus on va tendre vers un polymère de plus en plus ramifié.

Dans le cadre de l'invention, et sauf mention contraire, on entend par « polymère ramifié », un polymère comprenant de une à plusieurs ramifications, mais sans être réticulé. Un tel polymère est différent d'un polymère réseau.

Selon un autre aspect, le procédé selon l'invention, peut comprendre une étape de réaction d'un composé de formule (I) telle que définie ci-dessus, avec :

- un diol parmi le groupe constitué des alcane-diols, des polyalkylène-diols et des diols présentant l'une des formules (II) ou (III) telles que définies ci-dessus ;
- un diisocyanate de formule (O)CN-$A_3$-NC(O) dans laquelle $A_3$ est tel que définie ci-dessus,
  à une température comprise de 40°C à 100°C, de préférence à 60°C, dans un solvant. Selon un mode de réalisation particulier, le diol utilisé dans le procédé est un composé de formule (II) :

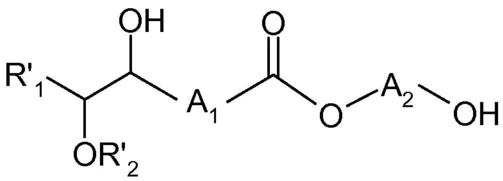

(II)

dans laquelle :

- $R'_1$ représente un groupe alkyle, linéaire ou ramifié, comprenant de 2 à 14 atomes de carbone ;
- $R'_2$ représente un groupe alkyle, linéaire ou ramifié, comprenant de 1 à 8 atomes de carbone ;
- $A_1$ représente un radical alkylène divalent, linéaire ou ramifié, comprenant de 2 à 14 atomes de carbone ;
- $A_2$ représente un radical alkylène divalent, linéaire ou ramifié, comprenant de 1 à 10 atomes de carbone.

Préférentiellement, le diol utilisé dans le procédé est un composé de formule (II) dans laquelle $R'_1$ représente un groupe octyle, $R'_2$ représente un groupe éthyle, $A_1$ représente un radical heptylène et $A'_2$ représente un radical pentylène.

Selon un mode de réalisation particulier, le diol utilisé dans le procédé est un composé de formule (III) :

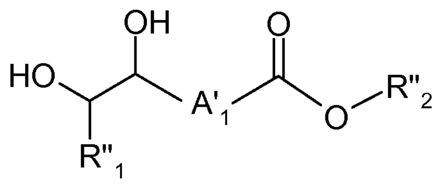

(III)

Préférentiellement, le diol utilisé dans le procédé est un composé de formule (III) dans laquelle $R''_1$ représente un groupe octyle, $R''_2$ représente un groupe éthyle et $A'_1$ représente un radical heptylène.

Selon un mode de réalisation, dans le procédé selon l'invention, lorsque R' représente un groupe (B) ou (B') tels que définis précédemment, le diol utilisé est de préférence, le diol de formule (III).

Selon un mode de réalisation, dans le procédé selon l'invention, lorsque R' représente un groupe (D) ou (D') tels que définis précédemment, le diol utilisé est de préférence, le diol de formule (II).

Selon un mode de réalisation, le solvant utilisé dans le procédé peut être choisi parmi les solvants permettant la solvatation du composé de formule (I) susmentionnée, notamment le DMF ou le DMSO, pour obtenir un polyuréthane réseau.

Selon un autre mode de réalisation, le solvant utilisé dans le procédé peut être choisi parmi les solvants ne permettant pas la solvatation du composé de formule (I), notamment le THF, pour obtenir un polyuréthane linéaire.

Ainsi il a été préparé des polymères, et notamment des polyuréthanes, biocompatibles, à partir de monomères biosourcés.

Il a été montré qu'il existe une sélectivité de la fonctionnalité des monomères glycosylés de l'invention, selon la nature du solvant utilisé dans le procédé. Une telle sélectivité permet de conduire de façon contrôlée à la fois à des polymères linéaires mais également à des polymères réseaux.

Ainsi, de nouveaux polyuréthanes présentant des propriétés physicochimiques tout à fait inattendues pour des polyuréthanes ont été préparés. En effet, les polyuréthanes selon l'invention présentent des propriétés physicochimiques, thermomécaniques et rhéologiques améliorées par rapport aux polyuréthanes issus de dérivés de corps gras connus de l'état de la technique. Ces derniers présentent généralement des propriétés thermomécaniques limitées, telles qu'une température de transition vitreuse basse et des modules faibles.

Les inventeurs ont avantageusement montré que l'incorporation des monomères glycolipides selon l'invention permet de moduler et d'améliorer les propriétés thermomécaniques, physicochimiques et rhéologiques des polyuréthanes résultants. Les polyuréthanes selon l'invention ont avantageusement des températures de transition vitreuse modulables de -40°C à +150°C.

Ainsi, l'incorporation de monomères glycolipides selon l'invention permet d'obtenir des polyuréthanes sous forme de solides beaucoup plus rigides que les polyuréthanes issus des corps gras qui sont sous forme d'huiles visqueuses.

Selon un autre aspect de l'invention, les polymères obtenus peuvent être utilisés pour la vectorisation, l'encapsulation ou la reconnaissance moléculaire, notamment dans le domaine médical, pharmaceutique ou cosmétique ; la séparation chromatographique notamment dans le domaine de l'analyse ; ou pour la préparation d'adhésifs, de cotensioactifs ou de revêtements. Selon l'invention, l'utilisation peut dépendre de la nature du polymère obtenu, à savoir s'il est linéaire, plus ou moins ramifié, ou réseau.

Selon un autre aspect, l'invention concerne des composés intermédiaires répondant à la formule suivante (I') :

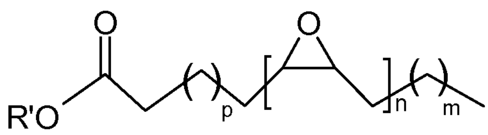

(I')

dans laquelle :

- R' est choisi parmi les sucres ou les sucre-alcools ;
- n est compris de 1 à 3 ;
- m est compris de 1 à 6 ; et
- p est compris de 1 à 9.

Selon un mode de réalisation, les composés intermédiaires de formule (I') sont choisis parmi les composés suivants :

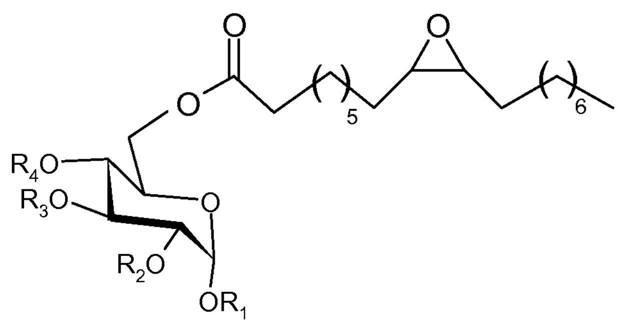

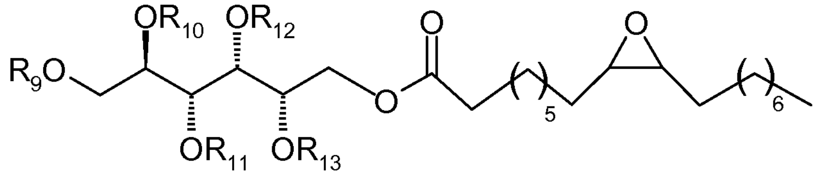

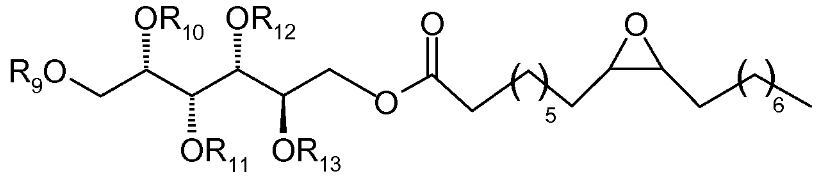

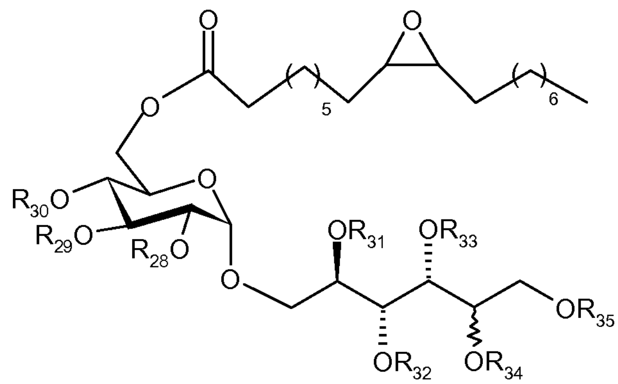

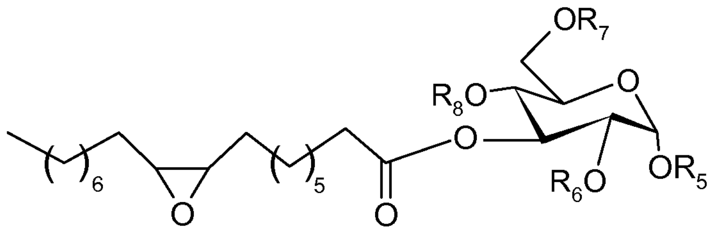

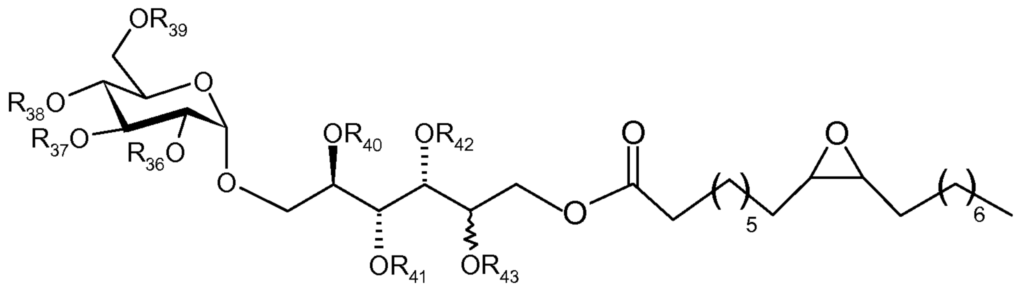

De préférence, les composés intermédiaires de formule (I') susmentionnée, sont choisis parmi les composés suivants :

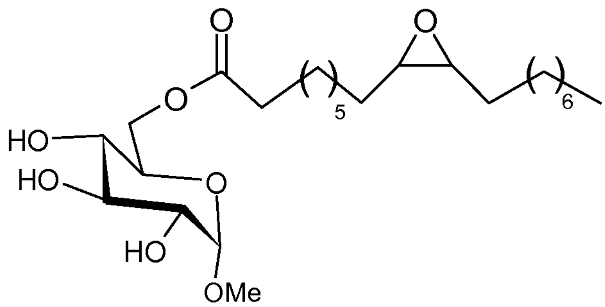

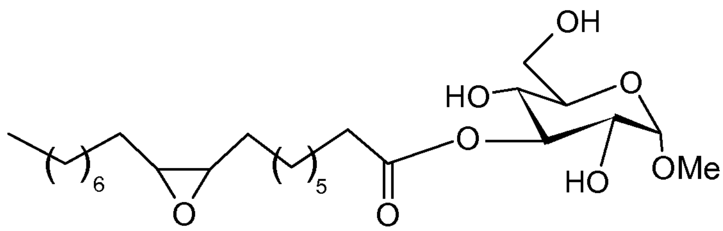

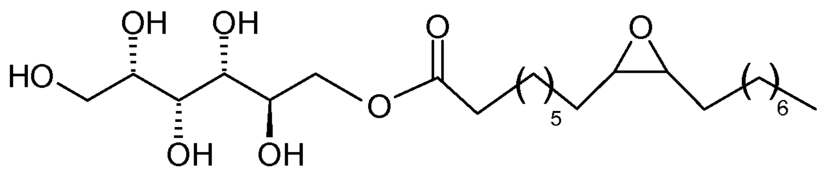

et

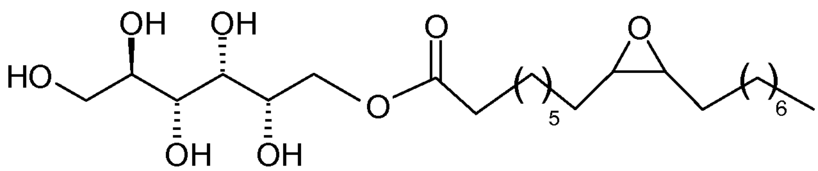

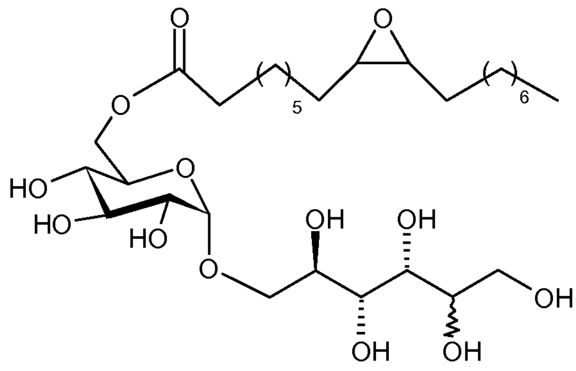

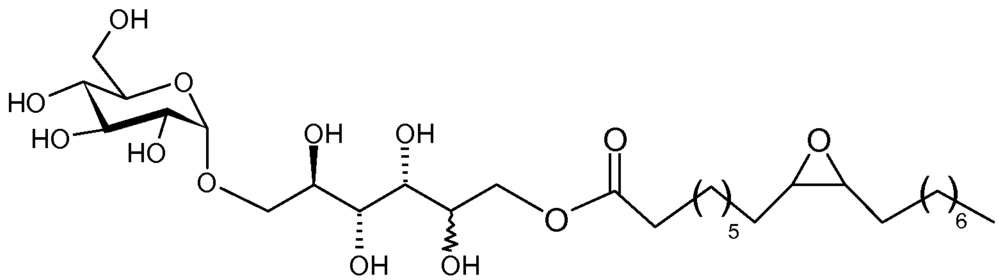

Selon l'invention, les composés de formule (I) peuvent être obtenus en deux étapes :

a) transestérification d'un composé de formule (V) suivante :

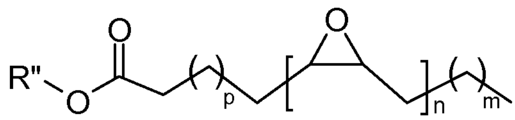

(V)

dans laquelle :

- R" représente un groupe alkyle comprenant de 1 à 10 atomes de carbone, de préférence de 1 à 5 atomes de carbone ;
- n est un entier compris de 1 à 3 ;
- m est un entier compris de 1 à 6 ; et
- p est un entier compris de 1 à 9.

avec un composé choisi parmi les sucres ou sucre-alcools suivants :

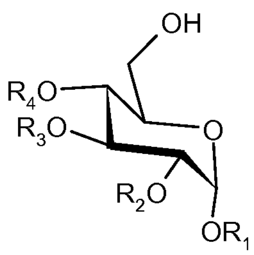

(B1)

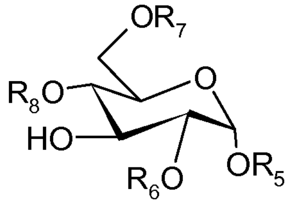

(B'1)

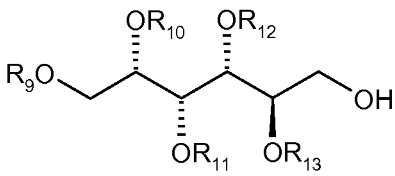

(C1)

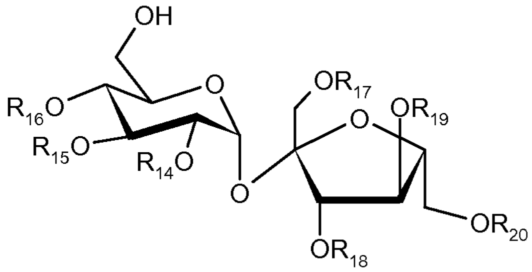

(D1)

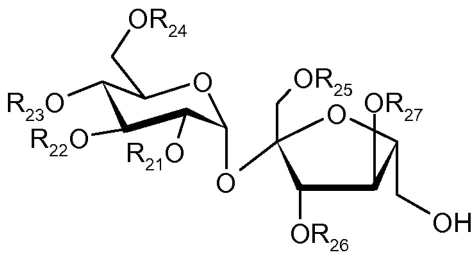

(D'1)

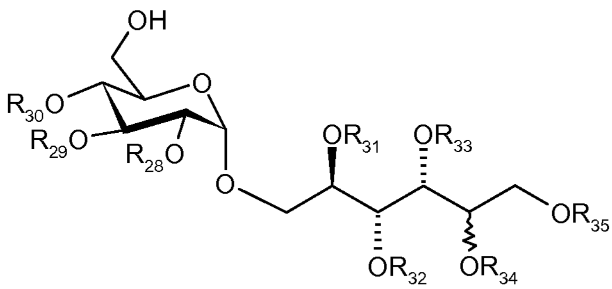

(E1)

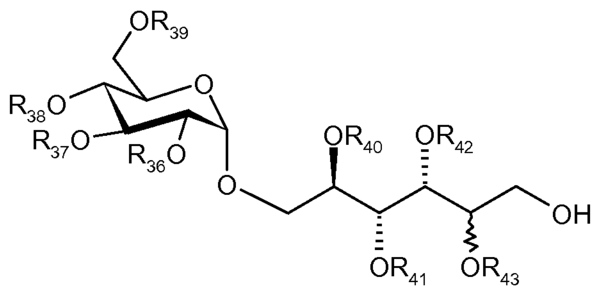

(E'1)

dans lesquels les groupements $R_1$ à $R_{43}$ sont tels que définis précédemment ; pour conduire aux composés intermédiaires de formule (I') ; et

b) une hydrolyse de la fonction époxyde pour conduire aux composés de formule (I) tel que définie précédemment.

De préférence, les composés préférés de formule (V) sont ceux pour lesquels n vaut 1 et R" représente méthyle ou éthyle.

Typiquement, la réaction de transestérification a) peut être réalisée dans tout type de solvant, de préférence ne pouvant pas réagir avec l'un des réactifs, en présence d'un catalyseur acide ou basique. De préférence, la réaction selon l'invention est réalisée dans le DMSO en présence de $K_2CO_3$ comme catalyseur basique.

Typiquement, la réaction a) peut être réalisée à une température comprise de 30°C à 100°C. De préférence, la réaction a) peut être effectuée de 50°C à 95°C, et préférentiellement de 60°C à 90°C.

Selon un mode de réalisation, l'étape d'hydrolyse b) peut être réalisée dans un solvant à une température comprise de 30°C à 100°C. De préférence, la réaction est réalisée dans le diméthylisosorbide (DMI) ou dans le DMSO, à une température comprise de 50°C à 90°C, et préférentiellement de 80°C à 90°C.

Typiquement, l'hydrolyse peut être réalisée en présence d'un acide choisi parmi l'acide chlorhydrique, l'acide sulfurique, l'acide nitrique et l'acide phosphorique. De préférence, l'hydrolyse b) est réalisée en présence d'acide phosphorique.

Selon un autre mode de réalisation, les composés de formule (I) peuvent être obtenus en une seule étape par une réaction de transestérification d'un composé de formule (VI) suivante :

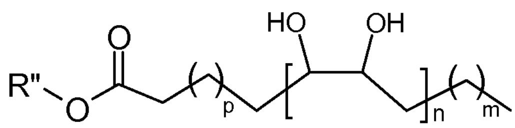

(VI)

dans laquelle :

- R" représente un groupe alkyle comprenant de 1 à 10 atomes de carbone, de préférence de 1 à 5 atomes de carbone ;
- n est un entier compris de 1 à 3 ;
- m est un entier compris de 1 à 6 ; et
- p est un entier compris de 1 à 9.

avec un composé choisi parmi les sucres ou sucre-alcools de formules (B), (B'), (C), (D), (D'), (E) ou (E') telles que définies précédemment, pour conduire aux produits de formule (I) telle que définie précédemment.

Les exemples suivants permettent d'illustrer l'invention sans toutefois la limiter.

**<u>EXEMPLES</u>**

***<u>Abréviations</u> :***

- AcOEt : Acétate d'éthyle ;
- AOG : 6-*O* methyl α-D-glucopyranosyl-9,10-dihydroxy octadecanoate (également appelé le 9,10-dihydroxystéarate de methyl α-D-glucopyranoside) (composé D) ;
- MeOH : Méthanol ;

- DAM : Dichlorométhane/Acétone/Méthanol
- DMI : Diméthylisosorbide ;
- CTAB : Bromure d'hexadécyltriméthylammonium ;
- IPDI : Isophorone diisocyanate ;
- DBTDL : Dilaurate de dibutylétain.

***<u>Fournisseurs</u> :***

| Types | Réactifs | Fournisseurs |
|---|---|---|
| Sucres | α-D-méthylglucoside | Sigma-Aldrich |
| | Sorbitol | Sigma-Aldrich |
| | Isomalt | Cargill Detschland GmbH |
| | Saccharose | Alfa-Aesar |
| Esters gras | Oléate de méthyle (99%)° | Aldrich |
| | Oléate d'éthyle (85%) | ITERG |
| | Oléate de méthyle époxydé (85%) | ITERG |
| Acides et Bases | $H_3PO_4$ (85%) | Carlo-Erba |
| | $K_2CO_3$ | Carlo-Erba |
| | $NH_4Cl$ | Carlo-Erba |
| | $NaHCO_3$ | Prolabo |
| | $HCO_2H$ | Prolabo |
| | m-CPBA | Aldrich |
| Solvants | DMSO | Carlo-Erba |
| | DMI | Roquettes |
| | $CH_2Cl_2$ | SDS |
| | Acétate d'éthyle | SDS |
| | *n*-Butanol | Sigma-Aldrich |
| | Acétonitrile | SDS |
| | $Na_2SO_4$ | SDS |
| | $H_2O_2$(35%) | Sigma-Aldrich |

A. **<u>SYNTHESE DES MONOMERES</u>**

**<u>Exemple 1 :</u> Préparation du 9,10-dihdroxy-octadécanoate de 6-*O*-α-D-méthylglucosyle (2)**

**1) Synthèse du 9,10-époxy-octadécanoate de méthyle (B)**

A 3,5g d'oléate de méthyle (11,8mmol ; 1éq.) et 0,18ml d'acide formique (4,7mmol ; 0,4éq), à 0°C, ont été ajoutés goutte à goutte 2,1mL d'eau oxygénée (35% poids. aq. ; 70,7mmol ; 6éq). Le milieu réactionnel a été agité pendant 4h à température ambiante puis à 40°C. Après 16h, la conversion totale de l'oléate de méthyle par CCM (Pentane/Acétate d'éthyle 98/2) a été observée. Après neutralisation à pH = 7 par une solution saturée en $NaHCO_3$, le mélange a été extrait avec de l'acétate d'éthyle (3 x 25mL). Les phases organiques ont été lavées avec 75mL d'une solution saturée en NaCl, séchées sur $Na_2SO_4$, filtrées puis évaporées à sec. Le composé **(B)** a été obtenu avec un rendement de 98% (huile jaune).

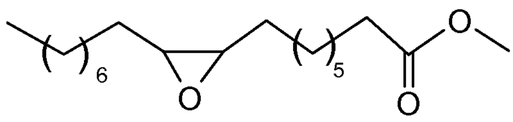

(B)

**2) Synthèse du 9,10-époxy-octadécanoate de 6-*O*-α-D-méthylglucosyle (1) et du 9,10-époxy-octadécanoate de 3-*O*-α-D-méthylglucosyle (1') par transestérification**

Dans un ballon de 250mL muni d'un barreau aimanté et d'un système de distillation, 10g d'oléate époxydé de méthyle (32mmol) **(B),** 4éq. de 1-*O*-méthyl-α-D-glucopyranoside (24g ; 128mmol) et 0,2éq de $K_2CO_3$ (0,9g ; 6,4mmol) ont été dissous dans 70mL de DMSO, et le milieu réactionnel a été laissé sous pression réduite (14-27mmHg) de 70°C à 90°C. La réaction a été suivie par CCM.

Après conversion totale de l'oléate époxydé de méthyle **(B),** le DMSO a été évaporé sous pression réduite. Le résidu a été solubilisé dans 300mL d'eau distillée et extrait avec de l'acétate d'éthyle (3 fois 250mL). Les phases organiques ont été lavées par 200mL d'une solution saturée en NaCl. La phase organique a été séchée sur $Na_2SO_4$, filtrée et l'acétate d'éthyle a été évaporé sous pression réduite. Le brut a été purifié par chromatographie sur silice normale avec un gradient de Pentane/AcOEt allant de 60/40 à 2/98 pour obtenir 11g d'un mélange de monoesters de méthylglucoside **(1)** et **(1')** (73%).

Le monoester en $C_6$ du méthylglucoside (1) a pu être isolé par précipitation dans un mélange Pentane/AcOEt : 80/20 à froid. 5,4g de poudre blanche peuvent être obtenus après filtration sur fritté (36% de rendement).

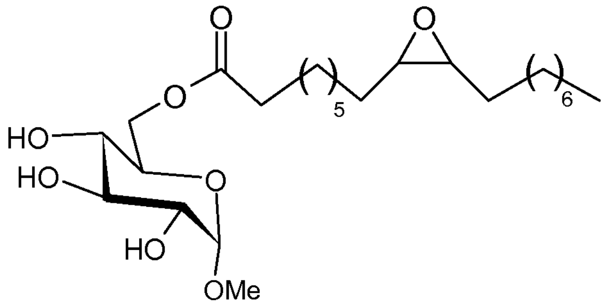

(1)

**Rf :** 0,34 (AcOEt/pentane : 9/1)

**RMN $^1$H (CDCl$_3$, 400MHz) :** 4,73 (d ; 1 H ; $J_{1-2}$ = 4,4Hz ; H-1); 4,34-4,40 (dd ; 1H, $J_{5-6a}$ = 6,7 Hz; $J_{6a-6b}$ = 15,2 Hz; H-6a) ; 4,26-4,33 (dd ; 1H; $J_{5-6b}$ = 6,7 Hz ; $J_{6a-6b}$ = 15,2 Hz; H-6b) ; 3,67-3,77 (m; 2H ; H-3 et H-5); 3,51 (m; H ; H-2); 3,40 (s; 3H; $OCH_3$ (méthylglucopyranoside)); 3,32 (t ; 1 H ; J=12,6 Hz; H-4) ; 2,88 (m ; 2H ; H-9' et H-10') ; 2,35 (t ; 2H ; J = 10,4 Hz ; $CH_2$ (α OCO)) ; 1,15-1,70 (m ; 26H ; $CH_2$ (chaîne alkyle)) ; 0,86 (t ; 3H ; J = 8,8 Hz; $CH_3$)

**RMN $^{13}$C (CDCl$_3$, 100MHz) :** 175,5 (C=O) ; 99,8 (C-1); 74,5 (C-3) ; 72,4 (C-2) ; 70,6 (C-4); 70,1 (C-5) ; 63,8 (C-6); 57,4 (C-9' et C-10'); 55,4 ($OCH_3$ (méthylglucopyranoside) ; 34,5 ($CH_2$ (α OCO)) ; 23,1-34,6 ($CH_2$ (chaîne alkyle)); 14,5 ($CH_3$)

**SM :** (haute résolution) m/z $[M+Na]^+$ masse calculée = 497,3085
masse mesurée = 497,3066

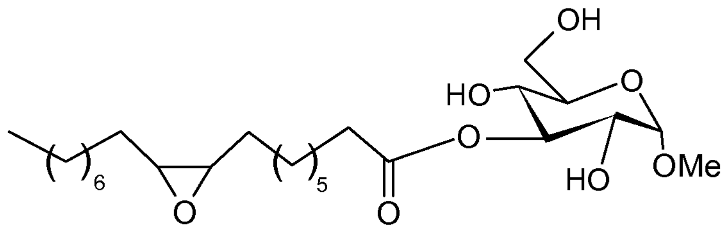

(1')

**Rf :** 0,55 (AcOEt/pentane : 9/1)

**RMN $^1$H (CDCl$_3$, 400MHz) :** 5,05 (t; 1H; $J_{3-4}$ = $J_{2-3}$9,2 Hz; H-3); 4,70 (d ; 1H ; $J_{1-2}$ = 3,7Hz ; H-1) ; 3,80-3,92 (m ; 2H; H-6a,b) ; 3,55-3,70 (m ; 3H ; H-2; H-4 et H-5) ; 3,40 (s; 3H; $OCH_3$ (méthylglucopyranoside)) ; 2,90 (m ; 2H ; H-9' et H-10') ; 2,41 (t ; 2H ; J = 10,4 Hz; $CH_2$ (α OCO)) ; 1,20-1,75 (m ; 26H ; $CH_2$ (chaîne alkyle)) ; 0,87 (t ; 3H ; J = 8,8

Hz; $CH_3$)

**RMN $^{13}C$ ($CDCl_3$, 100MHz) :** 176,0 (C=O) ; 99,7 (C-1) ; 77,6 (C-3) ; 71,8 (C-5) ; 71,1 (C-2) ; 69,6 (C-4) ; 62,6 (C-6); 57,7 (C-9' et C-10'); 55,8 ($OCH_3$ (méthylglucopyranoside) ; 34,5 ($CH_2$ (α OCO)) ; 23,06-32,2 ($CH_2$ (chaîne alkyle)); 14,5 ($CH_3$).

**SM :** (haute résolution) m/z $[M+Na]^+$ masse calculée = 497,3085
masse mesurée = 497,3066

**3) Synthèse du 9,10-dihydroxy-octadécanoate de 6-*O*-α-D-méthylglucosyle (2)**

Dans un ballon de 50mL muni d'un barreau aimanté, 1g de monoester d'oléate époxydé de méthylglucoside **(1)** (2,1mmol) ont été dissous dans 1,8mL de diméthylisosorbide (DMI) à 50°C, puis 5,6mL d'une solution de $H_3PO_4$ (5%massique dans l'eau) ont été rajoutés lentement et le milieu réactionnel a été porté à 80°C. La réaction a été suivie par CCM.

Après 3-4h de réaction, il y a eu conversion de **(1)** (par CCM). Le milieu réactionnel a été extrait avec de l'acétate d'éthyle (2fois 50mL) et les phases organiques ont été lavées avec une solution saturée en $NaHCO_3$ jusqu'à pH égale à 7 puis avec une solution saturée en NaCl (75mL). Les phases organiques ont été séchées sur $Na_2SO_4$, filtrées et l'acétate d'éthyle évaporé sous pression réduite. 15mL d'éther ont été ajoutés au résidu à 0°C. Un solide a précipité, puis il a été filtré pour récupérer le monoester diol oléate de méthylglucoside **(2)** (0,64g ; 62% de rendement).

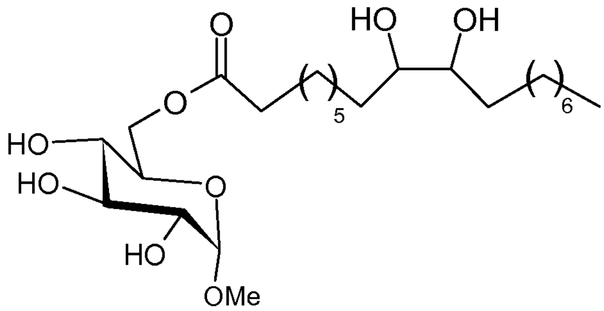

(2)

**Rf :** 0,41 (DAM : Dichlorométhane/Acétone/Méthanol : 8/1/1)

**RMN $^1H$ ($CD_3OD$, 400MHz)** : 4,73 (d ; 1 H ; $J_{1-2}$ = 4,4Hz ; H-1) ; 4,34-4,40 (dd ; 1H, $J_{5-6a}$ = 6,7 Hz; $J_{6a-6b}$ = 15,2 Hz; H-6a) ; 4,26-4,33 (dd ; 1H; $J_{5-6b}$ = 6,7 Hz; $J_{6a-6b}$ = 15,2 Hz; H-6b) ; 3,67-3,77 (m ; 2H ; H-3 et H-5) ; 3,64-3,71 (m ; 2H ; H-9' et H-10') ; 3,51 (m ; 2H ; H-2); 3,40 (s; 3H; $OCH_3$ (méthylglucopyranoside)); 3,32 (t ; 1 H ; J=12,6 Hz; H-4) ; 2,35 (t ; 2H ; J = 10,4 Hz ; $CH_2$ (α OCO)) ; 1,15-1,70 (m ; 26H ; $CH_2$ (chaîne alkyle)) ; 0,86 (t ; 3H ; J = 8,8 Hz; $CH_3$).

**RMN $^{13}C$ ($CD_3OD$, 100MHz) :** 175,5 (C=O); 101,6 (C-1); 75,6 (C-9' et C-10') ; 75,4 (C-3) ; 73,8 (C-2) ; 72,2 (C-4) ; 71,4 (C-5) ; 65,1 (C-6); 56,04 ($OCH_3$ (méthylglucopyranoside) ; 35,5 ($CH_2$ (α OCO)) ; 24,5-34,4 ($CH_2$ (chaîne alkyle)); 14,9 ($CH_3$).

**SM :** (haute résolution) m/z $[M+Na]^+$ masse calculée = 515,3191
masse mesurée = 515,3181

<u>Exemple 2 :</u> **Préparation du 9,10-dihydroxy-octadécanoate de 1-*O*-D-sorbitol (4) et 9,10-dihydroxy-octadécanoate de 6-*O*-D sorbitol (4')**

**1) Synthèse d'esters de sorbitol 9,10-époxy-octadécanoate de 1-*O*-D-sorbitol (3) et 9,10-époxy-octadécanoate de 6-*O*-D-sorbitol (3'), par transestérification**

Dans un ballon de 100mL muni d'un barreau aimanté et d'un système de distillation, 5g d'oléate de méthyle époxydé **(B)** (16 mmol), 4éq. de D-sorbitol (11,7g ; 64 mmol) et 0,2éq. de $K_2CO_3$ (0,4g ; 3,2 mmol) ont été dissous dans 35mL de DMSO, et le milieu réactionnel a été laissé sous pression réduite (14-27mmHg) de 70°C à 90°C. La réaction a été suivie par CCM. Après conversion totale de l'oléate de méthyle époxydé **(B),** le DMSO a été évaporé sous pression réduite. Le résidu a été solubilisé dans 150mL d'eau distillée et extrait avec de l'acétate d'éthyle (3 fois 150mL) et les phases organiques ont été lavées avec 200mL d'une solution saturée en NaCl. Les phases organiques ont été séchées sur $Na_2SO_4$, filtrées et l'acétate d'éthyle a été évaporé sous pression réduite. Le brut a été purifié par chromatographie sur silice normale avec un gradient de Pentane/AcOEt allant de 60/40 à 2/98 pour obtenir 7,2g d'un mélange de monoesters de sorbitol **(3)** et **(3')** sous l'aspect d'un gel partiellement solide. Les monoesters peuvent être purifiés par précipitation dans le méthanol à froid. 5,2g de solide brun après filtration sur fritté et lavage avec du pentane peuvent

être obtenus (70% de rendement).

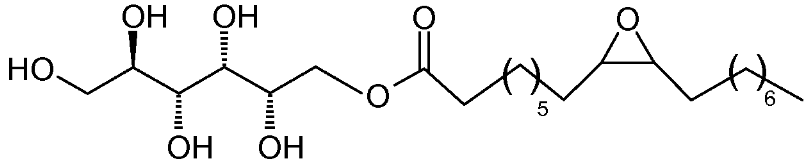

(3)

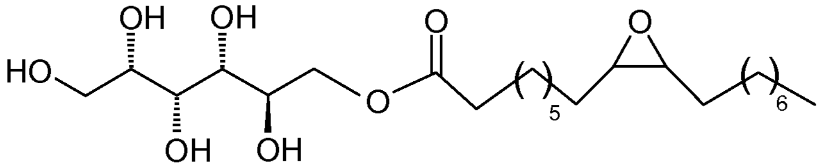

(3')

**Rf** 0.37 (AcOEt/MeOH 95/5)

**RMN $^1$H ($CD_3OD$, 400MHz) :** δ(ppm) 4.37 (dd, 1 H, *J* 15.2 Hz. *J* 3.5Hz. $COOCH_2$ A), 4.24 (dd, 1 H, *J* 15.2 Hz, *J* 1.5 Hz, $COOCH_2$ B), 4.18 (dd, 1 H, *J* 15.2 Hz, *J* 6.1 Hz, $COOCH_2$ B), 4.16 (dd, 1 H, *J* 15.2 Hz. *J* 3.5Hz. $COOCH_2$ A), 4.01-3.85 (m, 4H, C$\underline{H}$OH sorbitol), 3.82-3.59 (m, 8H, C$\underline{H}_2$OH A et B et C$\underline{H}$OH sorbitol), 2.95 (m, 4H, H-9' et H-10'), 2.39 (2t, 4H, J = 10.04Hz, $CH_2CO$), 1.31-1.65 (m , 52H , $CH_2$), 0.95 (t, 6H , J = 8.8Hz, $CH_3$).

**RMN $^{13}$C ($CD_3OD$, 125MHz):** δ(ppm) 175.8 et 175.5 (2xC=O), 75.1, 73.7, 73.5, 73.3, 73.0, 72.6, 70.9, 70.5(2), 67.4 (COO$\underline{C}H_2$ A), 66.7(COO$\underline{C}H_2$ B), 64.8 ($CH_2OH$ A or B), 64.1($CH_2OH$ A or B), 58.7 (2x C-9 et C-10 époxydes), 55.7 (2x$OCH_3$), 35.0 (2x$CH_2CO$), 23.7-33.0 ($CH_2$ chaîne alkyle), 14.4 (2x$CH_3$).

**SM :** (haute résolution) m/z $[M+Na]^+$ masse calculée = 485,3085
masse mesurée = 485,3063

**2) préparation du 9,10-dihydroxy-octadécanoate de 1-*O*-D-sorbitol (4) et 9,10-dihydroxy-octadécanoate de 6-*O*-D-sorbitol (4')**

a) Utilisation de DMI comme co-solvant

Dans un ballon de 100mL muni d'un barreau aimanté. 3,1g du mélange d'oléate époxydé de sorbitol **(3) et (3')** (6,7mmol) ont été dissous dans 4,1mL de diméthylisosorbide à 50°C, puis 11,5mL d'une solution de $H_3PO_4$ (5%massique dans l'eau) ont été rajoutés lentement. Le milieu réactionnel a été porté à 80-90°C et la réaction a été suivie par CCM. Après 5h de réaction, il y a eu conversion des époxydes **(3) et (3')** (par CCM) et le milieu réactionnel a été refroidi à température ambiante. Un gel jaune a précipité, puis été filtré et lavé à l'éther éthylique pour obtenir un solide jaune. Ce solide a été dissous dans 150mL d'acétate d'éthyle puis lavé 4 fois à l'eau distillée et avec une solution saturée de $NaHCO_3$, puis avec une solution saturée de NaCl. La phase organique a été séchée sur $Na_2SO_4$, filtrée et le solvant a été évaporé sous pression réduite. 2,3g de mélange de diol **(4)** et **(4')** (74%) contaminé par du DMI résiduel ont été obtenus.

b) Utilisation de DMSO comme co-solvant

Dans un ballon de 100mL muni d'un barreau aimanté, 2g du mélange d'époxydes **(3)** et **(3')** (4,1mmol) ont été dissous dans 4,1mL de DMSO à 50°C, puis 11,5mL d'une solution de $H_3PO_4$ (5%massique dans l'eau) ont été ajoutés lentement. Le milieu réactionnel a été porté à 80-90°C et la réaction a été suivie par CCM. Après 5h de réaction, il y a eu disparition de l'époxyde **(3)/(3')** (par CCM) et le milieu réactionnel a été laissé refroidir jusqu'à température ambiante. Un gel blanc a précipité, été filtré, et dissout dans 150mL d'acétate d'éthyle puis a été lavé 4 fois à l'eau distillée et avec une solution saturée de $NaHCO_3$, puis avec une solution saturée de NaCl. La phase organique a été séchée sur $Na_2SO_4$, filtrée et puis concentrée sous pression réduite. Une purification par précipitation à froid dans le diethyl éther a permis d'obtenir 1,8g de mélange de diols **(4)** et **(4')** (82%).

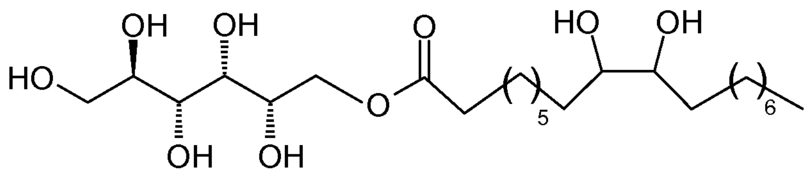

(4)

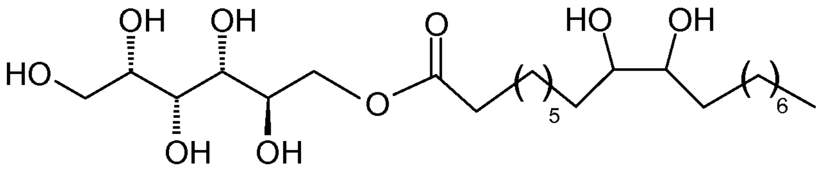

(4')

**Rf :** 0,44 (AcOEt/MeOH : 9/1)

**RMN $^1H$ ($CD_3OD$, 323K, 400MHz)** δ(ppm) 4.66 (dd , 1 H , *J* = 11.6Hz, *J* 2.8Hz, $COOCH_2$ A), 4.54-4.46(m, 2H, $COOCH_2$ B), 4.46 (dd, 1 H, *J* 11.7Hz, *J* 6.2Hz, $COOCH_2$ A), 4.28-4.12 (m, 4H, CHOH sorbitol), 4.10-3.89 (m, 8H, $CH_2OH$ A et B et CHOH sorbitol et CHOH sorbitol), 3.67(m, 4H, H-9' et H-10' dihydroxy), 2.66 and 2.65 (2t, 4H , J 7.5Hz, $2xCH_2CO$), 1.60-1.90 (m , 2x26H, $CH_2$), 1.19 (t, 6H , J 6.3Hz , $CH_3$).

RMN **$^{13}C$ ($CD_3OD$, 323K, 100MHz)** δ(ppm) 175.8 and 175.6 (2xC=O), 75.4 and 75.3 (CHOH 9,10-dihydroxy), 75.0, 73.8, 73.5, 73.2, 72.6, 71.1, 70.9(2), 67.4($COOCH_2$ A), 66.7($COOCH_2$ B), 64.9($CH_2OH$ A or B), 64.3 ($CH_2OH$ A or B), 35.1 and 35.0 ($CH_2CO$), 34.1-23.6 (aliphatique $CH_2$), 14.3($CH_3$).

**SM :** (haute résolution) m/z $[M+Na]^+$ masse calculée = 503,3191 masse mesurée = 503,3188

__Exemple 3 :__ **Préparation d'esters de saccharose 9,10-dihydroxy-octadécanoate de 6-*O*-saccharose (6) et (6')**

**1) Synthèse du 9,10-dihydroxy-octadécanoate de méthyle (C)**

Dans un ballon de 100mL muni d'un barreau aimanté, 1g d'oléate de méthyle époxydé **(B)** (3,1mmol) ont été dissous dans 5,8mL de diméthyl isosorbide (DMI), puis 8mL d'une solution de $H_3PO_4$ (5% massique dans l'eau) et 0,1g de bromure d'hexadécyltriméthylammonium (CTAB) (0,31mmol ; 0,1 éq.) on été ajoutés goutte à goutte. Puis, le milieu réactionnel a été porté à 80-90°C. Après la conversion de l'oléate de méthyle époxydé (par CCM), le mélange est refroidi à température ambiante pour 14h. Un précipité a été filtré sur fritté. Le solide a été solubilisé dans 100mL d'acétate d'éthyle et lavé avec l'eau distillée (6 fois 100mL) et avec une solution saturée en $NaHCO_3$ jusqu'à pH d'environ 7 puis avec une solution saturée en NaCl (2fois 50mL). La solution a été séchée sur $Na_2SO_4$, filtrée et évaporée. Le composé (C) a été obtenu avec un rendement de 86% (solide jaunâtre contenant du DMI résiduel).

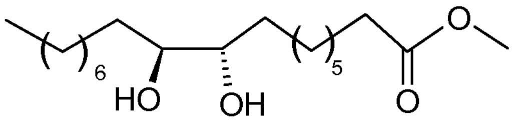

(C)

**Rf :** 0,27 (Acétate d'éthyle/pentane : 2/8)

**RMN $^1H$ ($CDCl_3$, 300MHz) :** 3,62 (s ; 3H ; $OCH_3$) ; 3,36-3,40(m; 2H ; H-9 et H-10); 2,26 (t ; 2H ; J = 7,5 ; $CH_2$ (α OCO)) ; 1,23-1,61 (m ; 26H ; $CH_2$ (chaîne alkyle)) ; 0,84 (t ; 3H ; J = 6,9 ; $CH_3$).

**RMN $^{13}C$ ($CDCl_3$, 75MHz) :** 174,2 (C=O) ; 75,3 (C-9; C-10) ; 51,7 ($C-OCH_3$) ; 34,1 ($CH_2$ (α OCO)) ; 22,7-31,9 ($CH_2$ (chaîne alkyle)); 14,1 ($CH_3$).

**SM :** (haute résolution) m/z $[M+Na]^+$ masse calculée =353,2662 masse mesurée =353,2657

**2) Synthèse de 9,10-dihydroxy-octadécanoate de 6 ou 6'-*O*-saccharose (6) et (6') par transestérification**

Dans un ballon de 500mL muni d'un barreau aimanté et d'un système de distillation, 18g de dihydroxy-oléate de méthyle **(C)** (83% en poids; 43,5mmol), 4éq. de saccharose (50,6g ; 147mmol) et 0,2éq de $K_2CO_3$ (1,02g ; 7,4mmol) ont été dissous dans 130mL de DMSO, et le milieu réactionnel a été agité sous pression réduite (14-27mmHg) de 70°C

à 90°C. Après conversion totale du diol **(C)** (CCM), le DMSO a été évaporé sous pression réduite. Le résidu a été solubilisé dans 250mL d'eau distillée et extrait d'abord avec de l'acétate d'éthyle (100mL) puis avec du butanol (3 fois 150mL) et les phases organiques ont été lavées avec 200mL de solution saturée en NaCl. Les phases organiques ont été séchées sur $Na_2SO_4$, filtrées et le butanol a été évaporé sous pression réduite (co-évaporé avec du méthanol pour enlever les traces de butanol). 6,3g de brut du mélange **(6)** et **(6')** ont été purifiés par chromatographie sur colonne avec un gradient d'élution de DAME-A à DAME-C pour obtenir 3,4g de monoesters de saccharose dont **(6)** et **(6').**

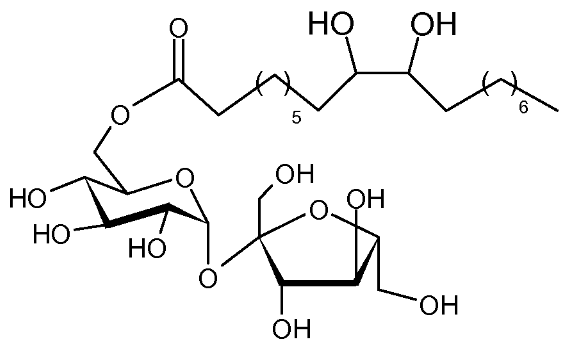

(6)

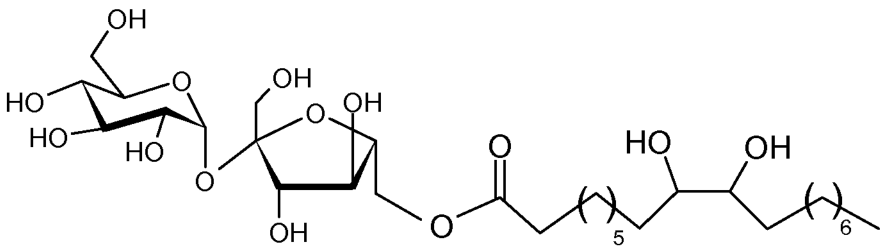

(6')

**Rf :** 0,30 (DAME-C)

L'analyse RMN du mélange après chromatographie est la suivante :

**RMN** $^1$H **($CD_3OD$; 400MHz)** (signaux principaux) : 5.36 (m, 2H, H-1sucrose), 4.43-4.40 (m, 3H, $\underline{C}H_2OCO$), 4.11-3.33 (m, H sucrose and 4H dihydroxy), 2.39 (t, 4H, *J* 10Hz, $CH_2CO$), 1.75-1.25 (m,2x26H, $CH_2$), 0.92 (t, 6H, *J* 8.8Hz, $CH_3$).

**RMN $^{13}$C ($CD_3OD$, 100MHz)** (signaux principaux) : 175.5 (C=O), 105.5, 105.2, 104.0 (C-2'sucrose), 93.5 and 93.4 (C-1 sucrose), 83.9-71.5 (CHOH sucrose and CHOH dihydroxy), 66.9 and 64.7 (2x $\underline{C}H_2OCO$), 64.1, 63.9, 63.8, 63.2, 62.5 (4x$OCH_2$), 35.1 and 34.9 (2x$\underline{C}H_2CO$), 35.1-23.7 ($CH_2$ aliphatique), 14.4 ($CH_3$).

**SM :** (haute résolution) m/z $[M+Na]^+$ masse calculée = 663,3562
masse mesurée = 663,3560

<u>Exemple 4 :</u> **Préparation d'esters d'isomalt (8) et (8')**

**1) Synthèse du 9,10-époxy-octadécanoate de 6-*O*-isomalt (7) et 9,10-époxy-octadécanoate de 6'-*O*-isomalt (7') par transestérification**

Dans un ballon de 500mL muni d'un barreau aimanté et d'un système de distillation, 10g d'oléate époxydé de méthyle **(B)** (30,6mmol), 4éq. d'isomalt (44g ; 128mmol) et 0,2éq de $K_2CO_3$ (0,8g ; 6,4mmol) ont été dissous dans 110mL de DMSO. Le milieu réactionnel a été agité sous pression réduite (14-27mmHg) de 70°C à 90°C. La réaction a été suivie par CCM. Après conversion totale d'oléate époxydé de méthyle **(B),** le DMSO a été évaporé sous pression réduite. Le résidu a été solubilisé dans 250mL d'eau distillée et extrait avec du butanol (3 fois 150mL) et les phases organiques ont été lavées avec 200mL de solution saturée en NaCl. Les phases organiques ont été séchées sur $Na_2SO_4$, filtrées, et le butanol a été évaporé sous pression réduite. 9g de brut ont été purifiés par chromatographie sur colonne avec un gradient d'élution de Dichlorométhane/Acetone/Methanol/Eau: 78/10/10/2 to 56/20/20/4 pour obtenir 5,2g de mélange contenant les monoesters d'isomalt **(7)** et **(7').**

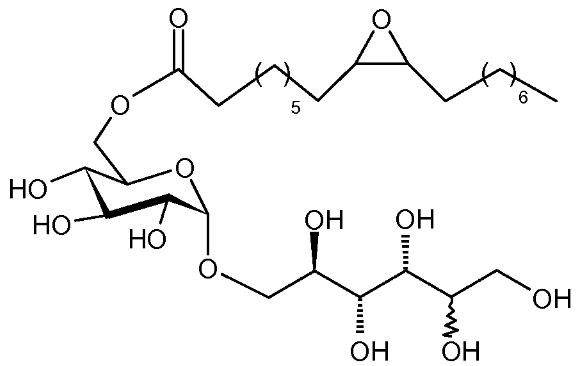

(7)

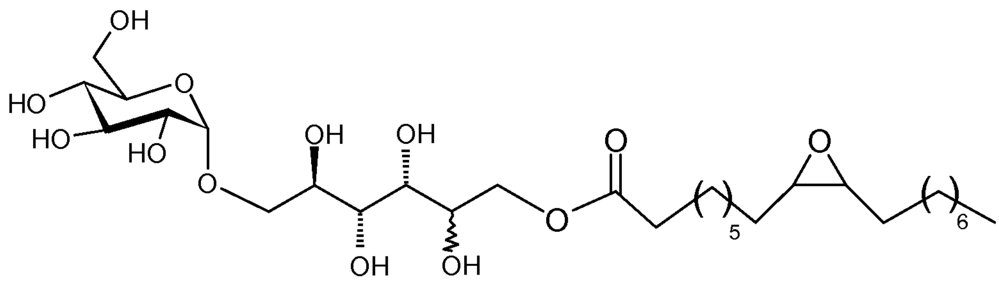

(7')

**Rf :** 0.28 (Dichlorométhane/Acetone/Methanol/Eau: 56/20/20/4)

L'analyse RMN ne permet d'identifier clairement les produits obtenus.
**SM :** (haute résolution) m/z $[M+Na]^+$ masse calculée = 647,3613
masse mesurée = 647,3612

**2) Synthèse de 9,10-dihydroxy-octadécanoate de 6-*O*-isomalt (8) et 9,10-dihydroxy-octadécanoate de 6'-*O*-isomalt (8') par hydrolyse**

Dans un ballon de 50mL muni d'un barreau aimanté, 1g du mélange d'oléate époxydé d'isomalt **(7)** et **(7')** (1,6mmol) a été dissous dans 3mL d'une solution de $H_3PO_4$ (5%massique dans l'eau) à 50°C. Ensuite, le milieu réactionnel a été porté à 80-90°C. La réaction a été suivie par CCM. Après 3-4h de réaction, il ya disparition de l'époxyde d'ester d'isomalt **(7)** (par CCM). Après avoir laissé le milieu revenir à température ambiante, un gel a précipité. Celui-ci a été récupéré par filtration. Le gel a été solubilisé dans 50mL de butanol, puis lavé avec une solution saturée en $NaHCO_3$ jusqu'à pH 7 puis avec une solution saturée en NaCl (2 fois 30mL). Les phases organiques ont été séchées sur $Na_2SO_4$, filtrées et le butanol a été évaporé sous pression réduite (co-évaporer avec du méthanol pour enlever les traces de butanol), pour obtenir 0,9g d'un mélange contenant les monoesters diol oléate d'isomalt **(8) et (8')** sous l'aspect d'un solide beige.

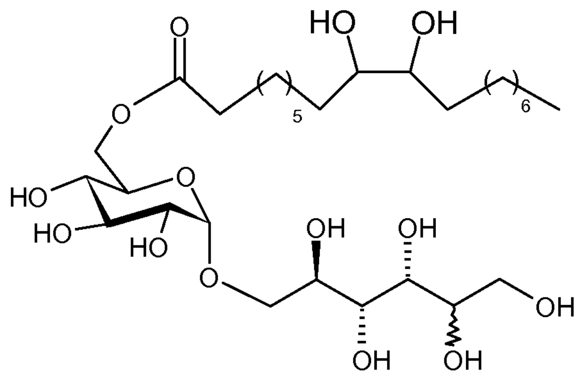

(8)

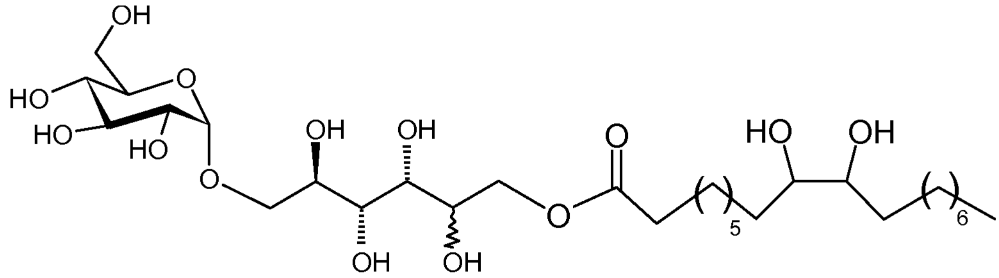

(8')

**Rf :** 0,17 (DAME-C: Dichlorométhane/Acetone/Methanol/Eau: 56/20/20/4)

L'analyse RMN ne permet pas d'identifier clairement les produits obtenus.
**SM :** (haute résolution) m/z $[M+Na]^+$ masse calculée = 665,3719
masse mesurée = 665,3697

**B. <u>SYNTHESE DE POLYURETHANES LINEAIRES A PARTIR DES MONOMERES</u>**

Le protocole appliqué décrit ci-après peut être appliqué à tout polyol glycosylé et tout isocyanate.

<u>Exemple 5 :</u> **Synthèse d'homopolymère à partir du 6-*O* methyl α-D-glucopyranosyl-9,10-dihydroxy octadecanoate (également appelé le 9,10-dihydroxystéarate de methyl α-D-glucopyranoside) (D) ou (AOG)**

**1) <u>Synthèse</u>**

Le composé **(D)** a été testé comme polyol dans la synthèse de nouveaux polyuréthanes, en présence d'IPDI.

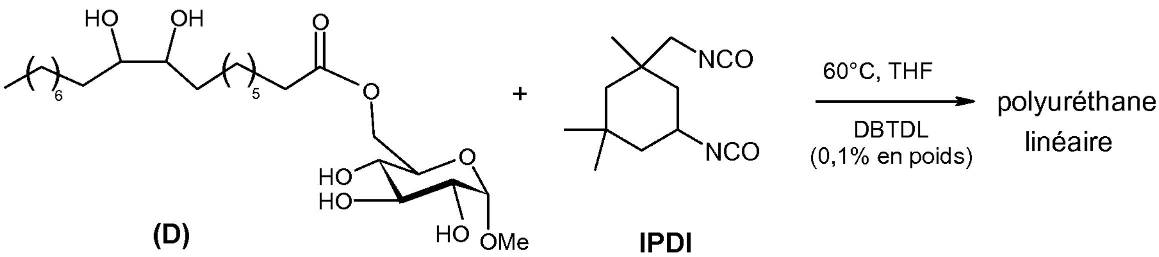


**(D)** **IPDI**

Dans un ballon de 250 mL surmonté d'un réfrigérant et muni d'un barreau aimanté ont été mélangés le composé **(D)** (5g ; 10,1 mmol) préalablement dissous dans 50 mL de THF, l'IPDI (isophorone diisocyanate) (2,47g; 11,1 mmol) et le catalyseur DBTDL (dilaurate de dibutylétain) (7,5 mg prélevés à la micropipette). Le ballon a ensuite été immergé dans un bain d'huile chauffé à 60°C. La conversion de l'IPDI a été suivie par spectroscopie infrarouge, grâce à la disparition de la bande de vibration des fonctions isocyanate localisées à 2250 $cm^{-1}$ ainsi que l'apparition de celles des fonctions uréthane localisée à 1530 $cm^{-1}$. Au bout de 20h, le polyuréthane a été obtenu.

Le Tableau 1 suivant rassemble les résultats des polymérisations réalisées dans le THF, pour différents rapports molaires fonctions isocyanate / fonctions alcool.

**Tableau 1 : Résultats expérimentaux des polymérisations réalisées à partir de du composé (D) et de l'IPDI dans le THF.**

| | Rapport molaire $n_{NCO}/n_{OH}$ | Durée réaction[a] | Conversion NCO | Solubilité polymère dans THF [b] | $M_w$ (g/mol)/IP [c] | Tg (°C) [d] |
|---|---|---|---|---|---|---|
| ***1*** | 1 | 20h | Incomplète | Insoluble | - | - |
| ***2*** | 0,7 | 20h | Incomplète | Insoluble | - | - |
| ***3*** | 0,5 | 20h | Incomplète | Insoluble | - | - |
| ***4*** | 0,38 | 20h | Totale | Soluble | 13 500 / 1,7 | 39 |

(suite)

| | Rapport molaire $n_{NCO}/n_{OH}$ | Durée réaction[a] | Conversion NCO | Solubilité polymère dans THF [b] | $M_w$ (g/mol)/IP [c] | Tg (°C) [d] |
|---|---|---|---|---|---|---|
| ***5*** | 0,2 | 20h | Totale | Soluble | 8 500 / 1,5 | 38 |

[a]*Déterminée lorsque la bande de vibration des fonctions isocyanate à 2250 $cm^{-1}$ n'évolue plus.*
[b] *Solubilité du polymère dans le solvant lorsque la réaction n'évolue plus.*
[c]*Déterminée par chromatographie SEC, solvant DMF, étalonnage PS, IP=dispersité=$M_w/M_n$.*
[d] *DSC, rampe de -100°C à 150°C, à 10°C/min.*

Pour des rapports molaires (fonctions isocyanates/fonctions alcools) compris entre 0,5 et 1 (entrées ***1, 2 et 3***), la conversion des fonctions isocyanate reste incomplète, ce qui se traduit par une absence de réactivité de certaines fonctions alcool.

Pour des rapports molaires inférieurs ou égaux à 0,38, la conversion des fonctions isocyanate est complète au bout de 20h. De façon remarquable, les polyuréthanes ainsi obtenus sont solubles dans le THF (entrées ***4*** et ***5*****).** Ces résultats ont montré que cette valeur seuil de 0,38 correspond à ne considérer que la seule contribution de seulement deux fonctions alcool sur les cinq que comporte le polyol **(D).**

L'analyse du spectre RMN $^1H$ du polyuréthane obtenu révèle la disparition du signal à 4,40 ppm des deux protons des carbones porteurs des fonctions alcool de la chaine C18. A l'inverse, les signaux localisés à 4,25 ppm, 4,50 ppm et 4,75 ppm relatifs aux protons des carbones porteurs des fonctions alcool du groupement glycoside n'ont pas disparu, ce qui indique que ces fonctions alcool n'ont pas réagi lors de la polymérisation.

La comparaison avec le spectre RMN $^{13}C$ du composé (D) montre que les fonctions alcool de la chaine C18 ont été consommées alors que celles du groupement glycoside ont été conservées. En effet, le signal à 73,17 ppm relatif aux deux carbones porteurs des alcools de la chaine C18 disparait à la fin de la polymérisation, alors que les pics à 73,08 ppm, 71,77 ppm et 69,55 ppm correspondants aux carbones du groupement glycoside sont encore présents.

Le fait que les deux fonctions alcool de la chaine C18 aient réagis prioritairement dans le THF s'explique par le fait que le glucose n'est pas soluble dans le THF, contrairement aux dérivés d'acide gras. La sélectivité de ces deux types de fonction alcool dans ce solvant permet donc d'obtenir des polyuréthanes linéaires pour un rapport molaire proche de 0,4.

Lors de l'utilisation d'un ratio fonctions isocyanates sur fonctions alcools de 0,2, on remarque qu'un polyuréthane linéaire a également été obtenu après une conversion totale. En revanche, la masse moléculaire obtenue est plus faible. Ceci s'explique par le fait que la totalité des fonctions hydroxyles de la partie chaîne grasse du polyol (D) n'ont pas réagi.

En revanche, lors de l'augmentation du ratio de 0,4 à 0,5, il en résulte que toutes les fonctions de la chaîne grasse ont réagi et que certaines fonctions hydroxyles de la partie sucre peuvent réagir. Il en résulte la formation de ramifications et donc l'obtention d'un polymère ramifié. Plus le ratio augmente et plus on va tendre vers un polymère réseau.

**2) <u>Propriétés thermomécaniques des polyuréthanes</u>**

La caractérisation des polyuréthanes par analyse enthalpique différentielle révèle des températures de transition vitreuse (Tg) proche de 38°C pour les polyuréthanes linéaires.

**<u>Exemple 6 :</u> Synthèse d'homopolymère à partir de l'oléate dérivé du saccharose**

L'ester de saccharose **(E)** a été testé comme polyol dans la synthèse de nouveaux polyuréthanes, en présence d'IPDI.

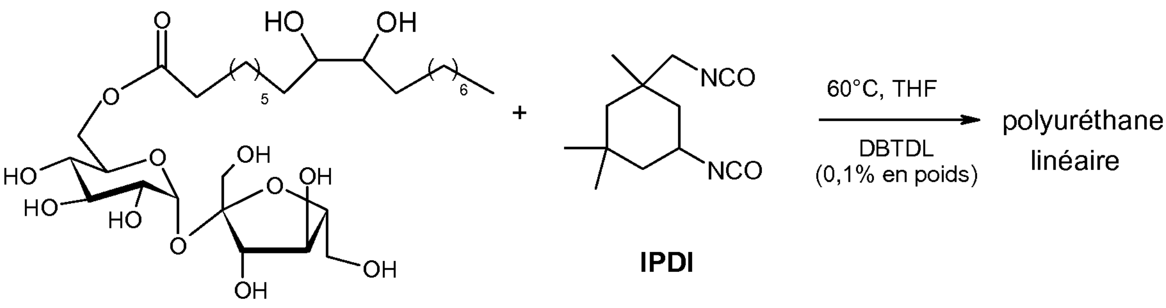


IPDI

(E)

La polymérisation a été effectuée selon le protocole décrit dans l'exemple 5. Le Tableau 2 suivant rassemble les résultats des polymérisations réalisées dans le THF, pour différents rapports molaires fonctions isocyanate / fonctions alcool (durée totale : 22h).

**Tableau 2 : Résultats expérimentaux des polymérisations réalisées à partir du saccharose et de l'IPDI dans le THF.**

| | (D) ($10^{-4}$ moles) | IPDI ($10^{-4}$ moles) | Rapport molaire $n_{NCO}/n_{OH}$ | DBTDL (en µL) | Conversion NCO | $M_n$ (g/mol)/IP | $T_g$ (en °C) |
|---|---|---|---|---|---|---|---|
| ***1*** | 7,5 | 7,5 | 1 | 0,66 | Totale | 21000-31200/2-2,25 | 32,6 et 110,9 |
| ***2*** | 7,5 | 3 | 0,4 | 0,56 | Totale | 9900/1,23600/1 | 18,8 et 69,8 |

Le polyuréthane est obtenu sous forme d'une poudre dans tous les cas.

**<u>Exemple 7:</u> Synthèse de copolymères composé (D)/OPH (Oléate de Pentanol Hydroxylé)**

**1) <u>Synthèse</u>**

Une série de copolymères ont été synthétisés à partir de l'oléate de pentanol hydroxylé **(F),** de composé **(D)** et en présence d'IPDI.

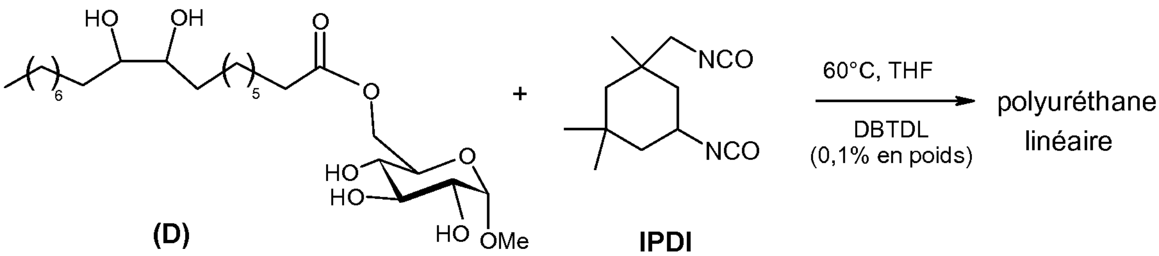


(D)

IPDI

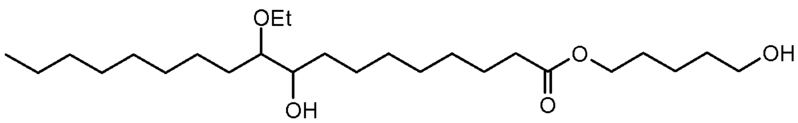

(F)

Les réactions de polymérisation ont été effectuées dans le THF. Seules les deux fonctions alcool de la chaine C18 du composé **(D)** et les deux fonctions alcool de l'OPH ont été prises en compte pour calculer la quantité d'IPDI à

ajouter. Le composé **(D)** a été incorporé à différentes fractions molaires variant de 0 à 100%. Les conversions totales ont été atteintes lorsque la bande de vibration des fonctions isocyanate a complètement disparu en infrarouge.

En considérant que la fraction molaire en AOG (composé **(D)**), incorporé dans le mélange de monomères AOG/OPH est $X_{AOG}$ et que la masse totale de diol est de 5g, alors la masse d'IPDI à introduire a été calculée de la manière suivante :

$$n_{IPDI} = x_{AOG}.n_{AOG} + [(1 - x]_{AOG}).n_{OPH} \quad (1)$$

$$m_{AOG} + m_{OPH} = 5 \quad (2)$$

$$(1) + (2) \rightarrow m_{IPDI} = \left[M_{IPDI}[x]_{AOG}.\frac{m_{AOG}}{M_{AOG}} + [(1 - x]_{AOG}).\frac{(5 - m_{AOG})}{M_{OPH}}\right]$$

Dans un ballon de 250 mL surmonté d'un réfrigérant et muni d'un barreau aimanté ont été incorporés 5g d'un mélange AOG /OPH (avec une fraction molaire $X_{AOG}$ en AOG) dissous dans 50 mL de THF, ainsi que l'IPDI dont la masse $m_{IPDI}$ a été calculée selon l'expression ci-dessus, et enfin le catalyseur DBTDL (0,1 % en masse par rapport à la masse totale des réactifs). Le ballon a été immergé dans un bain d'huile chauffé à 60°C de 1 à 10 heures. La conversion de l'IPDI a été suivie par spectroscopie Infrarouge, grâce à la disparition de la bande de vibration des NCO localisée à 2250 $cm^{-1}$.

Le tableau 5 rassemble les valeurs des masses molaires et des Tg des polyuréthanes linéaires obtenus.

**Tableau 5 : Masses molaires et $T_g$ des polyuréthanes linéaires AOH/OPH en fonction de la fraction molaire en AOG (composé D)**

| | Fraction molaire en AOG | $M_w$ (g/mol) [a] | $M_w/M_n$ | Tg (°C) [b] |
|---|---|---|---|---|
| ***1*** | 100% | 15 600 | 1,8 | 39 |
| ***2*** | 80% | 14 500 | 1,8 | 32 |
| ***3*** | 60% | 13 100 | 1,7 | 24 |
| ***4*** | 40% | 12 900 | 1,6 | 11 |
| ***5*** | 0% | 13 500 | 1,7 | -36 |

*[a] SEC, solvant DMF, étalonnage PS.*
*[b] DSC, rampe de -50°C à 100°C, 100°C/min.*

Les valeurs des masses molaires des copolymères AOG /OPH varient légèrement en fonction de la proportion en AOG (composé **(D)**), la plus grande (15 600 g/mol) étant obtenue pour l'homopolymère préparé à partir de l'OPH. Ceci peut s'expliquer par le fait que ce dernier possède une fonction alcool terminal plus réactive.

Les thermogrammes obtenus par DSC révèlent des Tg comprises de -36 à 39°C. L'évolution de la Tg des polyuréthanes linéaires en fonction de la proportion en composé **(D)** semble suivre une tendance polynomiale.

**<u>Exemple 8 :</u> Synthèse de copolymères à partir du monomère (E) et du diol (G)**

Une série de copolymères ont été synthétisés à partir du diol **(G)** et du monomère **(E)** synthétisé précédemment, et en présence d'IPDI.

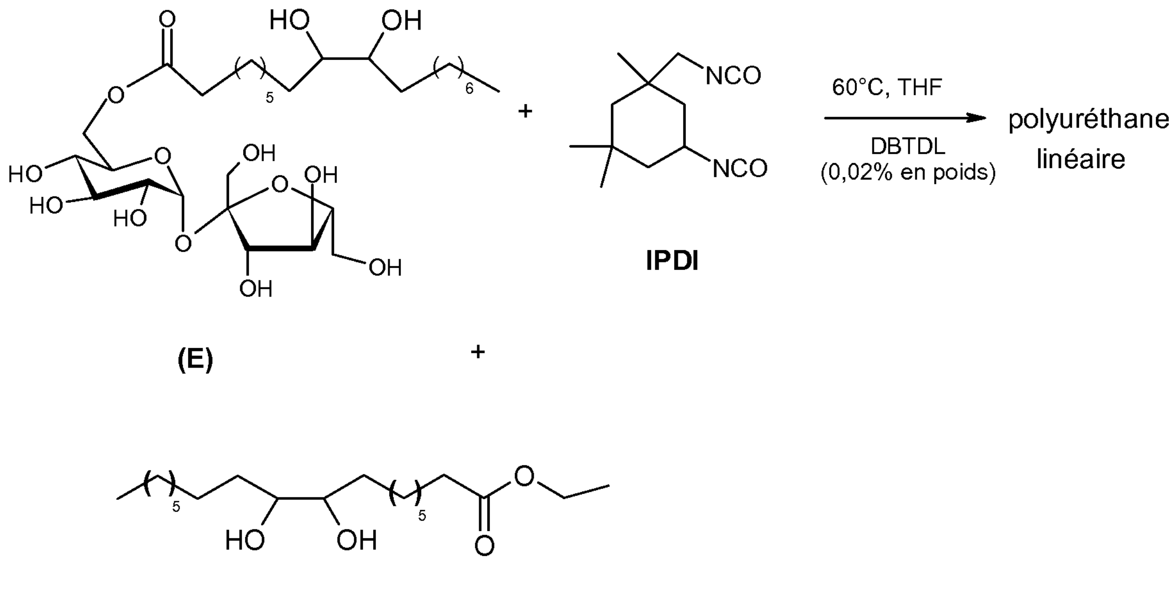


IPDI

(E) +

(G)

Différentes réactions de polymérisation ont été effectuées dans les conditions de l'exemple 7.

Le Tableau 6 suivant rassemble les résultats des polymérisations réalisées dans le THF, pour différents rapports massiques (durée totale : 22h).

**Tableau 6 : Résultats expérimentaux des polymérisations réalisées à partir du saccharose et de l'IPDI dans le THF.**

| | (E) ($10^{-4}$ moles) | IPDI ($10^{-3}$ moles) | (G) ($10^{-4}$ moles) | Rapport molaire $n_{NCO}/n_{OH}$ | DBTDL (en µL) | Composition en masse | Mn/IP |
|---|---|---|---|---|---|---|---|
| ***1*** | 2,8 | 1,97 | 16,9 | 1 | 0,2 | 25/75 | 2600/1,6 |
| ***2*** | 5,6 | 1,68 | 11,28 | 1 | 0,2 | 50/50 | 26800/- |

## C. SYNTHESE DE RESEAUX DE POLYURETHANES A PARTIR DES MONOMERES

***Exemple 9** : Synthèse d'homopolymère à partir du* **composé** ***(D)***

**1) Synthèse**

Le composé **(D)** a été testé comme polyol dans la synthèse de nouveaux polyuréthanes réseaux, en présence d'IPDI.

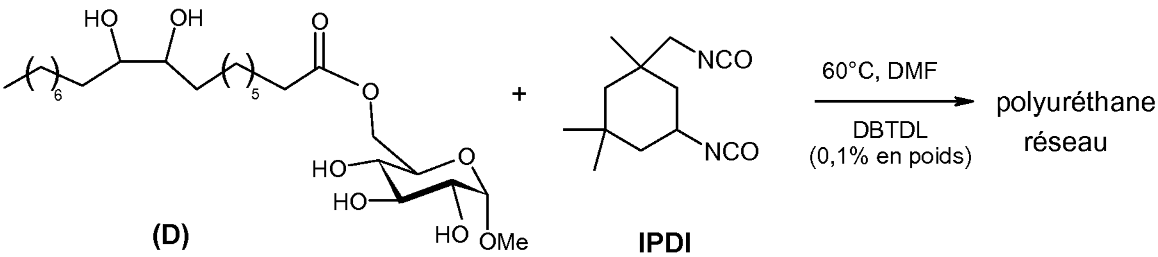


(D) IPDI

Dans un ballon de 250 mL surmonté d'un réfrigérant et muni d'un barreau aimanté a été mélangés le composé **(D)** (5g ; 10,1 mmol) préalablement dissout dans 50 mL de DMF, l'IPDI (5,61 g ; 25,3 mmol) et le catalyseur DBTDL (28 mg, 0,1% en masse par rapport à la masse totale des réactifs). Le ballon a ensuite été immergé dans un bain d'huile chauffé à 60°C. Au bout de 15h, le polyuréthane a été obtenu par évaporation du solvant.

Plusieurs essais de polymérisation ont été réalisés dans le DMF, qui s'avère être un bon solvant du composé

**(D).** Le Tableau 7 suivant rassemble les résultats obtenus lors des polymérisations effectuées dans ce solvant, pour différents rapports fonctions isocyanate / fonctions alcool.

**Tableau 7 : Résultats expérimentaux des polymérisations réalisées à partir du composé (D) et de l'IPDI dans le DMF.**

| | **Rapport molaire $n_{NCO}/n_{OH}$** | **Durée réaction [a]** | **Conversion NCO** | **Solubilité polymère dans DMF [b]** | **Tg (°C) [c]** |
|---|---|---|---|---|---|
| ***1*** | 1 | 15h | Totale | Insoluble | 149 |
| ***2*** | 0,7 | 15h | Totale | Insoluble | 99 |
| ***3*** | 0,5 | 15h | Totale | Insoluble | 69 |
| ***4*** | 0,2 | 15h | Totale | Insoluble | 35 |

*[a]Déterminée lorsque la bande de vibration des fonctions isocyanate à 2250 $cm^{-1}$ n'évolue plus.*
*[b] Solubilité du polymère dans le solvant lorsque la réaction n'évolue plus.*
*[c] DSC, rampe de -100°C à 200°C, 10°C/min.*

Toutes les polymérisations menées pour des rapports molaires variant de 0,2 à 1 conduisent à une conversion totale des fonctions isocyanate et à l'obtention de polymères insolubles dans le DMF. Ce résultat s'explique logiquement par l'entière réactivité des cinq fonctions alcool dans ce solvant.

**2) Propriétés thermomécaniques des polyuréthanes**

La caractérisation des polyuréthanes réseaux par analyse enthalpique différentielle révèle des températures de transition vitreuse $T_g$ allant de 35 à 149°C pour les réseaux 3D. Les propriétés thermiques des réseaux peuvent en effet être modulées selon la valeur du rapport molaire NCO/OH qui influe directement sur la densité du réseau. Les températures de transition vitreuse varient alors de 35°C pour un rapport molaire de 0,2 à 149°C pour un rapport molaire de 1 (tableau 7).

***Exemple 10:* Synthèse de copolymères réseaux AOG/OPH (Oléate de Pentanol Hydroxylé)**

**1) Synthèse**

Une série de copolymères ont été synthétisés à partir de l'oléate de pentanol hydroxylé **(F),** du composé **(D)** (AOG) et en présence d'IPDI.

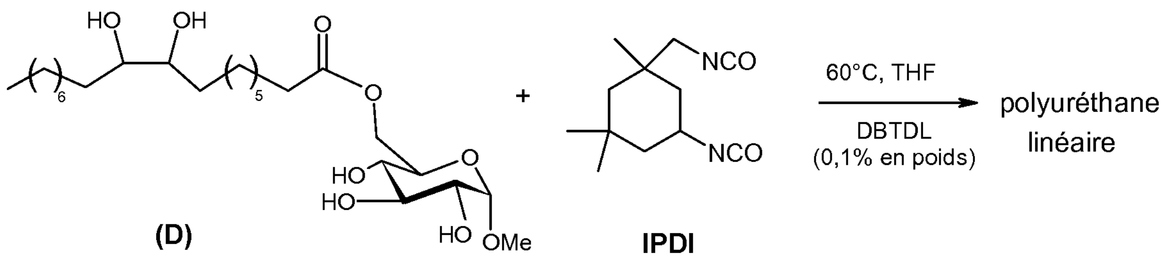


**(D)** **IPDI**

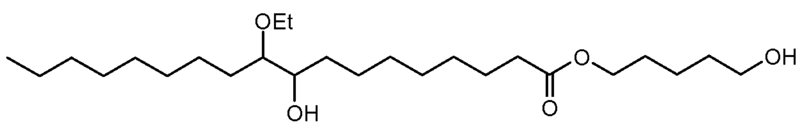

**(F)**

Les réactions de polymérisation ont été effectuées dans le DMF et l'IPDI a été introduit en proportion stoechiométrique par rapport au nombre totale de fonctions alcool. Les copolymères réseaux AOG/OPH ont été préparés à partir de différentes fractions molaires en AOG (composé **(D)**).

En considérant que la fraction molaire en AOG incorporé dans le mélange de monomères AOG/OPH est $X_{AOG}$

et que la masse totale de diol est de 5g, alors la masse d'IPDI à introduire a été calculée de la manière suivante :

$$n_{IPDI} = \frac{5}{2[x_{AOG}.n_{AOG} + [(1-x]_{AOG}).n_{OPH}]} \quad (1)$$

$$m_{AOG} + m_{OPH} = 5 \quad (2)$$

$$(1) + (2) \rightarrow m_{IPDI} = [\frac{5.M_{IPDI}}{2[x]_{AOG}}.\frac{m_{AOG}}{M_{AOG}} + [(1-x]_{AOG}).\frac{(5-m_{AOG})}{M_{OPH}}]$$

Dans un ballon de 250 mL surmonté d'un réfrigérant et muni d'un barreau aimanté a été incorporés 5g d'un mélange AOG/OPH (avec une fraction molaire $X_{AOG}$ en AOG) dissous dans 50 mL de DMF, ainsi que l'IPDI et le catalyseur DBTDL (0,1 % en masse par rapport à la masse totale des réactifs). Le ballon a été immergé dans un bain d'huile chauffé à 60°C.

**2) <u>Propriétés thermomécaniques des polyuréthanes réseaux</u>**

Le Tableau 5 suivant rassemble les valeurs des Tg des polyuréthanes obtenus.

**Tableau 5 : $T_g$ des polyuréthanes linéaires AOH/OPH en fonction de la fraction molaire en AOG (composé D).**

| | Fraction molaire en AOG | Tg (°C) [a] |
|---|---|---|
| ***1*** | 100% | 149 |
| ***2*** | 80% | 119 |
| ***3*** | 60% | 91 |
| ***4*** | 40% | 28 |
| ***5*** | 20% | -8 |
| ***6*** | 0% | -36 |

[a] *DSC, rampe de -50°C à 100°C, 10°C/min.*

L'analyse par AED des polyuréthanes résultants révèle clairement l'influence du composé **(D)** sur la Tg du matériau, celle-ci augmentant linéairement avec la fraction molaire en composé **(D).** Elle varie de -36°C pour un taux en composé **(D)** de 0% à 149°C pour 100% de composé **(D)** incorporé.

Ce résultat est très intéressant car il permet l'accès à des réseaux de polyuréthanes biosourcés présentant des propriétés thermomécaniques modulables.

## Revendications

**1.** Composé de formule (I) :

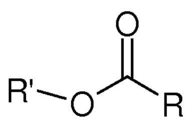
(I)

dans laquelle :

- R représente un groupe alkyle linéaire ou ramifié, comprenant de 8 à 27 atomes de carbone, ledit groupe alkyle étant substitué par au moins deux groupes hydroxyle, et pouvant éventuellement contenir une insaturation ; et
- R' est choisi parmi les sucres ou les sucre-alcools, notamment parmi le groupe constitué du thréose, de

l'érythrose, du désoxyribose, du ribose, du xylose, du ribulose, du lyxose, du glucose, du méthyl glucoside, du mannose, du fructose, de l'idose, du sorbose, du galactose, de l'allose, du maltose, du lactose, de l'isomaltose, de l'isomaltulose, du cellobiose, du saccharose, du raffinose, du mélézitose, du sorbitol, de l'isomalt, du xylitol, du mannitol et de l'arabinitol.

**2.** Composé selon la revendication 1, dans lequel R répond à la formule (A) :

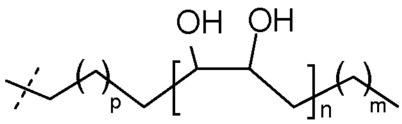

dans laquelle :

- n est compris de 1 à 3 ;
- m est compris de 1 à 6 ;
- p est compris de 1 à 9.

**3.** Composé selon l'une quelconque des revendications 1 ou 2, **caractérisé en ce que** R' répond à l'une des formules suivantes :

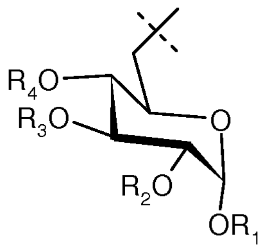
(B)

dans laquelle $R_1$, $R_2$, $R_3$ et $R_4$ représentent, indépendamment les uns des autres : H ; un groupe alkyle comprenant de 1 à 12 atomes de carbone ; un groupe $CH_3C(O)$- ; un groupe arylalkyle comprenant de 6 à 12 atomes de carbones ; un groupe ((C1-C6)alkyle)$_3$-Si- ou $R_1$ et $R_2$, $R_2$ et $R_3$ , ou $R_3$ et $R_4$, forment ensemble un groupe isopropylidène ;
ou

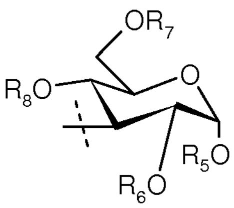
(B')

dans laquelle $R_5$, $R_6$, $R_7$ et $R_8$ représentent, indépendamment les uns des autres : H ; un groupe alkyle comprenant de 1 à 12 atomes de carbone ; un groupe $CH_3C(O)$- ; un groupe arylalkyle comprenant de 6 à 12 atomes de carbones; un groupe ((C1-C6)alkyle)$_3$-Si- ou $R_5$ et $R_6$ ou $R_7$ et $R_8$, forment ensemble un groupe isopropylidène ;
ou

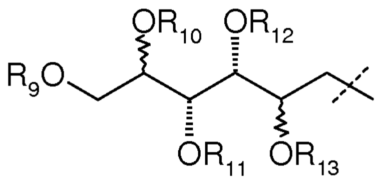

(C)

dans laquelle Rg, $R_{10}$, $R_{11}$, $R_{12}$ et $R_{13}$ représentent, indépendamment les uns des autres : H ; un groupe alkyle comprenant de 1 à 12 atomes de carbone ; un groupe $CH_3C(O)$- ; un groupe arylalkyle comprenant de 6 à 12 atomes de carbones ; un groupe ((C1-C6)alkyle)$_3$-Si- ou Rg et $R_{10}$, ou $R_{10}$ et $R_{11}$, ou $R_{11}$ et $R_{12}$, ou $R_{12}$ et $R_{13}$, forment ensemble un groupe isopropylidène ;
ou

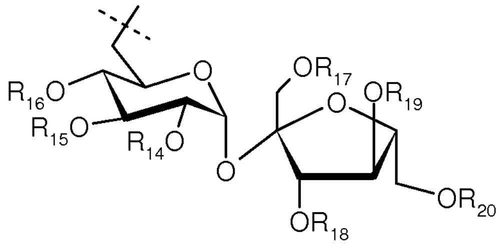

(D)

dans laquelle $R_{14}$, $R_{15}$, $R_{16}$, $R_{17}$, $R_{18}$, $R_{19}$ et $R_{20}$ représentent, indépendamment les uns des autres : H ; un groupe alkyle comprenant de 1 à 12 atomes de carbone ; un groupe $CH_3C(O)$- ; un groupe arylalkyle comprenant de 6 à 12 atomes de carbones ; un groupe ((C1-C6)alkyle)$_3$-Si- ou $R_{14}$ et $R_{17}$ ou $R_{19}$ et $R_{20}$ forment ensemble un groupe isopropylidène ;
ou

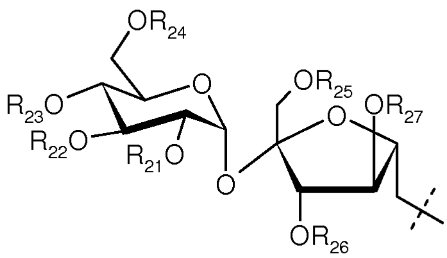

(D')

dans laquelle $R_{21}$, $R_{22}$, $R_{23}$, $R_{24}$, $R_{25}$, $R_{26}$ et $R_{27}$ représentent, indépendamment les uns des autres : H ; un groupe alkyle comprenant de 1 à 12 atomes de carbone ; un groupe $CH_3C(O)$- ; un groupe arylalkyle comprenant de 6 à 12 atomes de carbones ; un groupe ((C1-C6)alkyle)$_3$-Si- ou $R_{23}$ et $R_{24}$ ou $R_{21}$ et $R_{25}$ ou $R_{26}$ et $R_{27}$ forment ensemble un groupe isopropylidène.

4. Composé selon l'une quelconque des revendications 1 ou 2, **caractérisé en ce que** R' répond à l'une des formules suivantes :

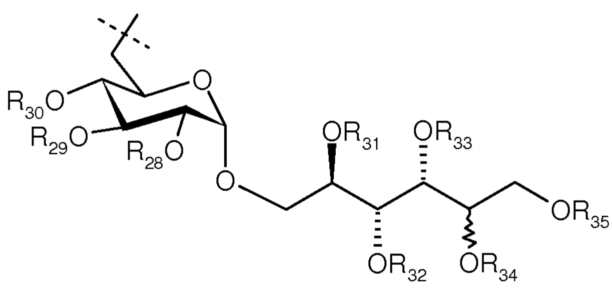

. (E)

dans laquelle $R_{28}$, $R_{29}$, $R_{30}$, $R_{31}$, $R_{32}$, $R_{33}$, $R_{34}$ et $R_{35}$ représentent, indépendamment les uns des autres : H ; un groupe alkyle comprenant de 1 à 12 atomes de carbone ; un groupe $CH_3C(O)$- ; un groupe arylalkyle comprenant de 6 à 12 atomes de carbones ; un groupe ((C1-C6)alkyle)$_3$-Si- ou $R_{29}$ et $R_{30}$ ou $R_{31}$ et $R_{32}$ ou $R_{32}$ et $R_{33}$ ou $R_{33}$ et $R_{34}$ forment ensemble un groupe isopropylidène ;
ou

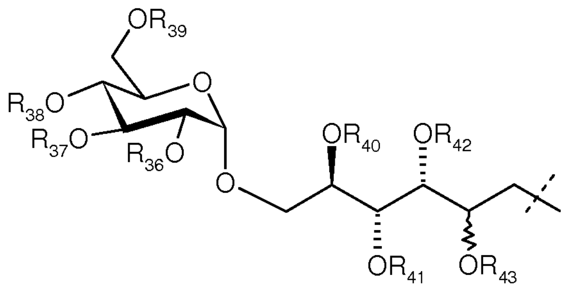

(E')

dans laquelle $R_{36}$, $R_{37}$, $R_{38}$, $R_{39}$, $R_{40}$, $R_{41}$, $R_{42}$ et $R_{43}$ représentent, indépendamment les uns des autres : H ; un groupe alkyle comprenant de 1 à 12 atomes de carbone ; un groupe $CH_3C(O)$- ; un groupe arylalkyle comprenant de 6 à 12 atomes de carbones ; un groupe ((C1-C6)alkyle)$_3$-Si- ou $R_{37}$ et $R_{38}$ ou $R_{38}$ et $R_{39}$ ou $R_{40}$ et $R_{41}$ ou $R_{41}$ et $R_{42}$, ou $R_{42}$ et $R_{43}$ forment ensemble un groupe isopropylidène.

**5.** Polymère susceptible d'être obtenu par polymérisation d'un composé tel que défini selon l'une quelconque des revendications 1 à 4 et d'un (poly)isocyanate, notamment d'un diisocyanate répondant à la formule (O)CN-$A_3$-NC(O), dans laquelle $A_3$ représente :

- un groupe alkylène, linéaire ou ramifié, comprenant de 2 à 20 atomes de carbone ; ou
- un groupe cycloalkylène-alkylène-cycloalkylène, comprenant de 6 à 30 atomes de carbone ; ou
- un groupe arylène-alkylène-arylène, comprenant de 6 à 30 atomes de carbone ; ou
- un groupe cycloalkylène, comprenant de 3 à 10 atomes de carbone ;
- un groupe alkylène-cycloalkylène, comprenant de 3 à 15 atomes de carbone ; ou
- un groupe arylène comprenant de 6 à 10 atomes de carbone.

**6.** Polymère susceptible d'être obtenu par polymérisation d'un composé tel que défini selon l'une quelconque des revendications 1 à 4, d'un diol et d'un (poly)isocyanate, notamment d'un diisocyanate répondant à la formule (O)CN-$A_3$-NC(O), dans laquelle $A_3$ représente :

- un groupe alkylène, linéaire ou ramifié, comprenant de 2 à 20 atomes de carbone ; ou
- un groupe cycloalkylène-alkylène-cycloalkylène, comprenant de 6 à 30 atomes de carbone ; ou
- un groupe arylène-alkylène-arylène, comprenant de 6 à 30 atomes de carbone ; ou
- un groupe cycloalkylène, comprenant de 3 à 10 atomes de carbone ;
- un groupe alkylène-cycloalkylène, comprenant de 3 à 15 atomes de carbone ; ou
- un groupe arylène comprenant de 6 à 10 atomes de carbone.

**7.** Polymère selon la revendication 6, **caractérisé en ce que** le diol est choisi parmi les alcane-diols, les polyalkylène-diols, les polyether-diols, les polyester-diols et les diols de formules suivantes :

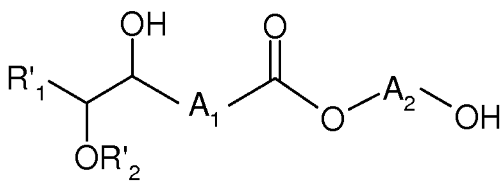

(II)

dans laquelle :

- $R'_1$ représente un groupe alkyle, linéaire ou ramifié, comprenant de 2 à 14 atomes de carbone ;

- $R'_2$ représente un groupe alkyle, linéaire ou ramifié, comprenant de 1 à 8 atomes de carbone ;
- $A_1$ représente un radical alkylène divalent, linéaire ou ramifié, comprenant de 2 à 14 atomes de carbone ;
- $A_2$ représente un radical alkylène divalent, linéaire ou ramifié, comprenant de 1 à 10 atomes de carbone ; et

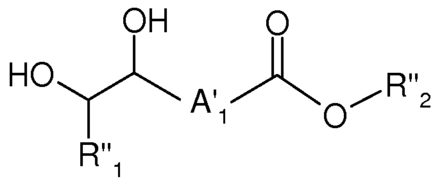

(III)

dans laquelle :

- $R''_1$ représente un groupe alkyle, linéaire ou ramifié, comprenant de 2 à 14 atomes de carbone ;
- $R''_2$ représente un groupe alkyle, linéaire ou ramifié, comprenant de 1 à 8 atomes de carbone ;
- $A'_1$ représente un radical alkylène divalent, linéaire ou ramifié, comprenant de 2 à 14 atomes de carbone.

**8.** Polymère selon la revendication 5, **caractérisé en ce que** le polymère répond à la formule suivante :

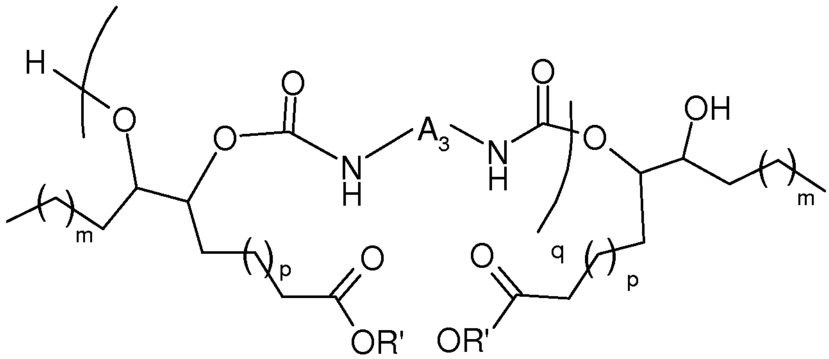

dans laquelle :

- n est 1 ;
- m est compris de 1 à 6 ;
- p est compris de 1 à 9 ;
- R' est choisi parmi les sucres ou les sucre-alcools ;
- $A_3$ est choisi parmi le groupe constitué de :

  - un groupe alkylène, linéaire ou ramifié, comprenant de 2 à 20 atomes de carbone ;
  - un groupe cycloalkylène-alkylène-cycloalkylène, comprenant de 6 à 30 atomes de carbone ;
  - un groupe arylène-alkylène-arylène, comprenant de 6 à 30 atomes de carbone ;
  - un groupe cycloalkylène, comprenant de 3 à 10 atomes de carbone ;
  - un groupe alkylène-cycloalkylène, comprenant de 3 à 15 atomes de carbone ; et
  - un groupe arylène comprenant de 6 à 10 atomes de carbone ;

- q représente un nombre entier compris entre 2 à 500 000, et de préférence de 2 à 50000, notamment de 2 à 5000, et encore de préférence de 2 à 50.

**9.** Procédé de préparation de polyuréthane, comprenant l'étape de réaction d'un composé selon l'une quelconque des revendications 1 à 4 avec un diisocyanate de formule (O)CN-$A_3$-NC(O), selon la revendication 6, à une température comprise de 40°C à 100°C, de préférence à 60°C, dans un solvant.

**10.** Procédé de préparation de polyuréthane, comprenant l'étape de réaction d'un composé selon l'une quelconque des revendications 1 à 4, avec :

- un diol ;
- un diisocyanate de formule (O)CN-$A_3$-NC(O) dans laquelle $A_3$ est choisi parmi le groupe constitué de :

    - un groupe alkylène, linéaire ou ramifié, comprenant de 2 à 20 atomes de carbone ;
    - un groupe cycloalkylène-alkylène-cycloalkylène, comprenant de 6 à 30 atomes de carbone ;
    - un groupe arylène-alkylène-arylène, comprenant de 6 à 30 atomes de carbone ;
    - un groupe cycloalkylène, comprenant de 3 à 10 atomes de carbone ;
    - un groupe alkylène-cycloalkylène, comprenant de 3 à 15 atomes de carbone ; et
    - un groupe arylène comprenant de 6 à 10 atomes de carbone ;

à une température comprise de 40°C à 100°C, de préférence à 60°C, dans un solvant.

**11.** Procédé selon la revendication 9 ou 10, **caractérisé en ce que** le solvant est choisi parmi les solvants permettant la solvatation du composé selon l'une quelconque des revendications 1 à 4, notamment le DMF ou le DMSO, pour obtenir un polyuréthane réseau.

**12.** Procédé selon la revendication 9 ou 10, **caractérisé en ce que** le solvant est choisi parmi les solvants ne permettant pas la solvatation du composé selon l'une quelconque des revendications 1 à 4, notamment le THF, pour obtenir un polyuréthane linéaire.

**13.** Utilisation des polymères selon l'une quelconque des revendications 5 à 8, pour la vectorisation, l'encapsulation ou la reconnaissance moléculaire, notamment dans le domaine médical, pharmaceutique ou cosmétique ; la séparation chromatographique notamment dans le domaine de l'analyse ; ou pour la préparation d'adhésifs, de co-tensioactifs ou de revêtements.

**14.** Composé intermédiaire répondant à la formule suivante (I') :

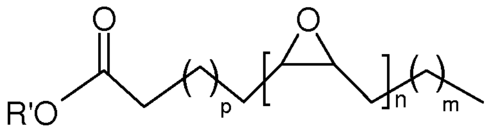

(I')

dans laquelle :

- R' est choisi parmi les sucres ou les sucre-alcools ;
- n est compris de 1 à 3 ;
- m est compris de 1 à 6 ; et
- p est compris de 1 à 9.

## Patentansprüche

**1.** Eine Verbindung der Formel (I):

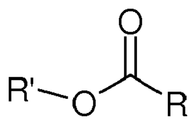

(I)

wobei:

- R eine lineare oder verzweigte Alkylgruppe darstellt, umfassend 8 bis 27 Kohlenstoffatome, wobei die Alkylgruppe mit mindestens zwei Hydroxylgruppen substituiert ist und wahlweise einfach ungesättigt ist; und
- R' ausgewählt ist aus Zuckern oder Zuckeralkoholen, insbesondere aus der Gruppe bestehend aus Threose, Erythrose, Desoxyribose, Ribose, Xylose, Ribulose, Lyxose, Glucose, Methylglycosid, Mannose, Fructose, Idose, Sorbose, Galactose, Allose, Maltose, Lactose, Isomaltose, Isomaltulose, Cellobiose, Saccharose, Raffinose, Melezitose, Sorbitol, Isomalt, Xylitol, Mannitol und Arabinitol.

**2.** Verbindung gemäß Anspruch 1, wobei R der Formel (A) entspricht:

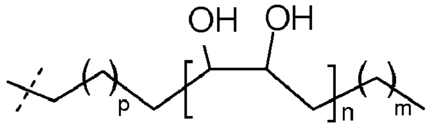

wobei:

- n 1 bis 3 ist;
- m 1 bis 6 ist;
- p 1 bis 9 ist.

**3.** Verbindung gemäß einem der Ansprüche 1 oder 2, **gekennzeichnet dadurch, dass** R einer der folgenden Formeln entspricht:

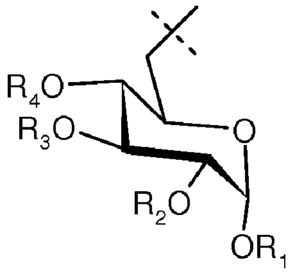

(B)

wobei $R_1$, $R_2$, $R_3$ und $R_4$ unabhängig voneinander H, eine Alkylgruppe umfassend 1 bis 12 Kohlenstoffatome; eine $CH_3C(O)$-Gruppe; eine Arylalkylgruppe umfassend 6 bis 12 Kohlenstoffatome; eine $((C1\text{-}C6)Alkyl)_3$-Si-Gruppe darstellen, oder $R_1$ und $R_2$, $R_2$ und $R_3$ oder $R_3$ und $R_4$ zusammen eine Isopropylidengruppe bilden;
oder

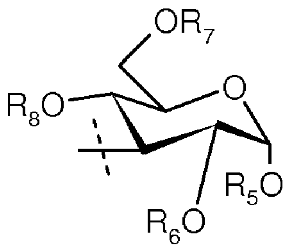

(B')

wobei $R_5$, $R_6$, $R_7$ und $R_8$ unabhängig voneinander H, eine Alkylgruppe umfassend 1 bis 12 Kohlenstoffatome; eine $CH_3C(O)$-Gruppe; eine Arylalkylgruppe umfassend 6 bis 12 Kohlenstoffatome; eine $((C1\text{-}C6)Alkyl)_3$-Si-Gruppe darstellen, oder $R_5$ und $R_6$ oder $R_7$ und $R_8$ zusammen eine Isopropylidengruppe bilden;
oder

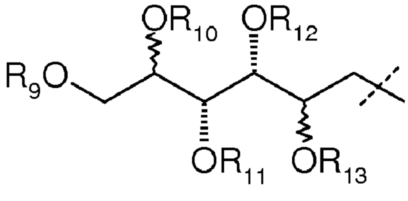

(C)

wobei $R_9$, $R_{10}$, $R_{11}$, $R_{12}$ und $R_{13}$ unabhängig voneinander H, eine Alkylgruppe umfassend 1 bis 12 Kohlenstoffatome; eine $CH_3C(O)$-Gruppe; eine Arylalkylgruppe umfassend 6 bis 12 Kohlenstoffatome; eine $((C1-C6)Alkyl)_3$-Si-Gruppe darstellen, oder $R_9$ und $R_{10}$, oder $R_{10}$ und $R_{11}$, oder $R_{11}$ und $R_{12}$, oder $R_{12}$ und $R_{13}$ zusammen eine Isopropylidengruppe bilden;
oder

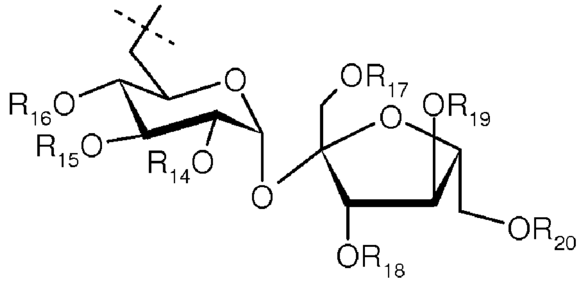

(D)

wobei $R_{14}$, $R_{15}$, $R_{16}$, $R_{17}$, $R_{18}$, $R_{19}$ und $R_{20}$ unabhängig voneinander H, eine Alkylgruppe umfassend 1 bis 12 Kohlenstoffatome; eine $CH_3C(O)$-Gruppe; eine Arylalkylgruppe umfassend 6 bis 12 Kohlenstoffatome; eine $((C1-C6)Alkyl)_3$-Si-Gruppe darstellen, oder $R_{14}$ und $R_{17}$, oder $R_{19}$ und $R_{20}$ zusammen eine Isopropylidengruppe bilden;
oder

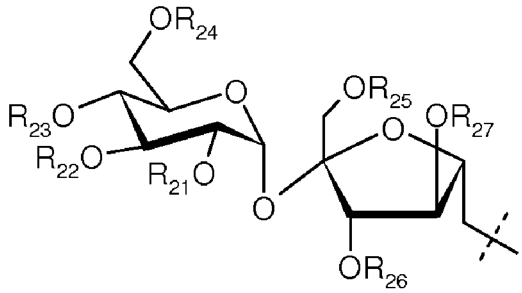

(D')

wobei $R_{21}$, $R_{22}$, $R_{23}$, $R_{24}$, $R_{25}$, $R_{26}$ und $R_{27}$ unabhängig voneinander H, eine Alkylgruppe umfassend 1 bis 12 Kohlenstoffatome; eine $CH_3C(O)$-Gruppe; eine Arylalkylgruppe umfassend 6 bis 12 Kohlenstoffatome; eine $((C1-C6)Alkyl)_3$-Si-Gruppe darstellen, oder $R_{23}$ und $R_{24}$ oder $R_{21}$ und $R_{25}$ oder $R_{26}$ und $R_{27}$ zusammen eine Isopropylidengruppe bilden.

4. Verbindung gemäß einem der Ansprüche 1 oder 2, **gekennzeichnet dadurch, dass** R' einer der folgenden Formeln entspricht:

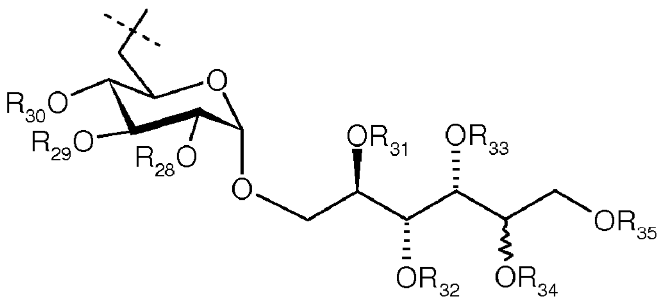

(E)

wobei $R_{28}$, $R_{29}$, $R_{30}$, $R_{31}$, $R_{32}$, $R_{33}$, $R_{34}$ und $R_{35}$ unabhängig voneinander H, eine Alkylgruppe umfassend 1 bis 12 Kohlenstoffatome; eine $CH_3C(O)$-Gruppe; eine Arylalkylgruppe umfassend 6 bis 12 Kohlenstoffatome; eine $((C1-C6)Alkyl)_3$-Si-Gruppe darstellen, oder $R_{29}$ und $R_{30}$ oder $R_{31}$ und $R_{32}$ oder $R_{32}$ und $R_{33}$ oder $R_{33}$ und $R_{34}$ zusammen eine Isopropylidengruppe bilden;

oder

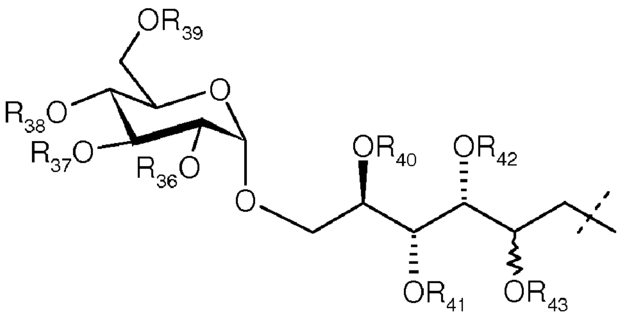

(E')

wobei $R_{36}$, $R_{37}$, $R_{38}$, $R_{39}$, $R_{40}$, $R_{41}$, $R_{42}$ und $R_{43}$ unabhängig voneinander H, eine Alkylgruppe umfassend 1 bis 12 Kohlenstoffatome; eine $CH_3C(O)$-Gruppe; eine Arylalkylgruppe umfassend 6 bis 12 Kohlenstoffatome; eine ((C1-C6)Alkyl)$_3$-Si-Gruppe darstellen, oder $R_{37}$ und $R_{38}$, oder $R_{38}$ und $R_{39}$, oder $R_{40}$ und $R_{41}$, oder $R_{41}$ und $R_{42}$, oder $R_{42}$ und $R_{43}$ zusammen eine Isopropylidengruppe bilden.

5. Polymer erhältlich durch Polymerisation einer Verbindung definiert gemäß einem der Ansprüche 1 bis 4 und eines (Poly)isocyanats, insbesondere eines Diisocyanats entsprechend der Formel (O)CN-$A_3$-NC(O), wobei $A_3$:

- eine lineare oder verzweigte Alkylengruppe umfassend 2 bis 20 Kohlenstoffatome; oder
- eine Cycloalkylen-Alkylen-Cycloalkylen-Gruppe umfassend 6 bis 30 Kohlenstoffatome; oder
- eine Arylen-Alkylen-Arylen-Gruppe umfassend 6 bis 30 Kohlenstoffatome; oder
- eine Cycloalkylengruppe umfassend 3 bis 10 Kohlenstoffatome;
- eine Alkylen-Cycloalkylen-Gruppe umfassend 3 bis 15 Kohlenstoffatome; oder
- eine Arylengruppe umfassend 6 bis 10 Kohlenstoffatome darstellt.

6. Polymer erhältlich durch Polymerisation einer Verbindung definiert gemäß einem der Ansprüche 1 bis 4, eines Diols und eines (Poly)isocyanats, insbesondere eines Diisocyanats entsprechend der Formel (O)CN-$A_3$-NC(O), wobei $A_3$:

- eine lineare oder verzweigte Alkylengruppe umfassend 2 bis 20 Kohlenstoffatome; oder
- eine Cycloalkylen-Alkylen-Cycloalkylen-Gruppe umfassend 6 bis 30 Kohlenstoffatome; oder
- eine Arylen-Alkylen-Arylen-Gruppe umfassend 6 bis 30 Kohlenstoffatome; oder
- eine Cycloalkylengruppe umfassend 3 bis 10 Kohlenstoffatome;
- eine Alkylen-Cycloalkylen-Gruppe umfassend 3 bis 15 Kohlenstoffatome; oder
- eine Arylengruppe umfassend 6 bis 10 Kohlenstoffatome darstellt.

7. Polymer gemäß Anspruch 6, **gekennzeichnet dadurch, dass** das Diol ausgewählt ist aus Alkandiolen, Polyalkylendiolen, Polyetherdiolen, Polyesterdiolen und Diolen der folgenden Formeln:

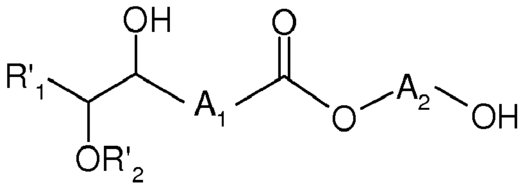

(II)

wobei:

- $R'_1$ eine lineare oder verzweigte Alkylgruppe umfassend 2 bis 14 Kohlenstoffatome darstellt;
- $R'_2$ eine lineare oder verzweigte Alkylgruppe umfassend 1 bis 8 Kohlenstoffatome darstellt;
- $A_1$ ein lineares oder verzweigtes, divalentes Alkylenradikal umfassend 2 bis 14 Kohlenstoffatome darstellt;
- $A_2$ ein lineares oder verzweigtes, divalentes Alkylenradikal umfassend 1 bis 10 Kohlenstoffatome darstellt;

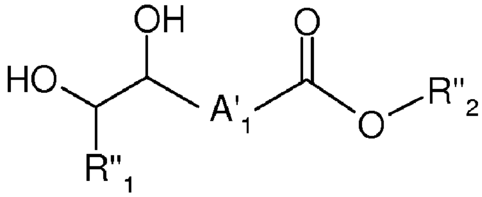

(III)

und
wobei:

- $R''_1$ eine lineare oder verzweigte Alkylgruppe umfassend 2 bis 14 Kohlenstoffatome darstellt;
- $R''_2$ eine lineare oder verzweigte Alkylgruppe umfassend 1 bis 8 Kohlenstoffatome darstellt;
- $A_1$ ein lineares oder verzweigtes, divalentes Alkylenradikal umfassend 2 bis 14 Kohlenstoffatome darstellt.

8. Polymer gemäß Anspruch 5, **gekennzeichnet dadurch, dass** das Polymer der folgenden Formel entspricht:

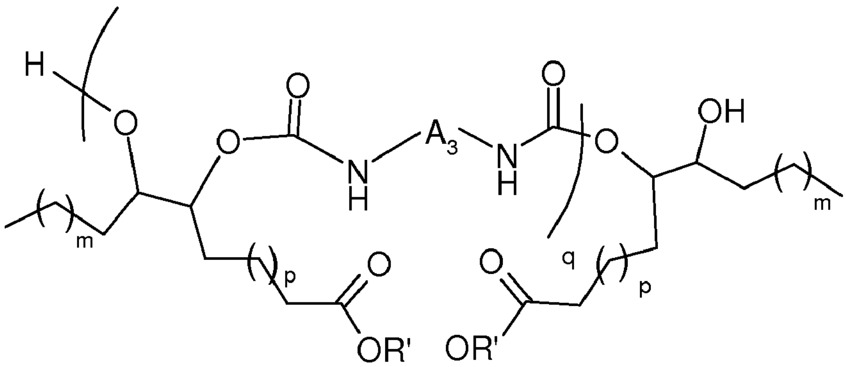

wobei:

- n 1 ist;
- m 1 bis 6 ist;
- p 1 bis 9 ist;
- R' ausgewählt ist aus Zuckern oder Zuckeralkoholen;
- $A_3$ ausgewählt ist aus der Gruppe bestehend aus:
- einer linearen oder verzweigten Alkylengruppe umfassend 2 bis 20 Kohlenstoffatome;
- einer Cycloalkylen-Alkylen-Cycloalkylen-Gruppe umfassend 6 bis 30 Kohlenstoffatome;
- einer Arylen-Alkylen-Arylen-Gruppe umfassend 6 bis 30 Kohlenstoffatome;
- einer Cycloalkylengruppe umfassend 3 bis 10 Kohlenstoffatome;
- einer Alkylen-Cycloalkylen-Gruppe umfassend 3 bis 15 Kohlenstoffatome; und
- einer Arylengruppe umfassend 6 bis 10 Kohlenstoffatome;
- q eine ganze Zahl zwischen 2 bis 500.000 darstellt, vorzugsweise von 2 bis 50.000, insbesondere von 2 bis 5000 und noch bevorzugter von 2 bis 50.

9. Verfahren zur Herstellung eines Polyurethans, umfassend den Schritt des Umsetzens einer Verbindung gemäß einem der Ansprüche 1 bis 4 mit einem Diisocyanat der Formel (O)CN-$A_3$-NC(O), gemäß Anspruch 6, bei einer Temperatur von 40 °C bis 100 °C, vorzugsweise bis 60 °C, in einem Lösemittel.

10. Verfahren zur Herstellung eines Polyurethans, umfassend den Schritt des Umsetzens einer Verbindung gemäß einem der Ansprüche 1 bis 4 mit:

- einem Diol;
- einem Diisocyanat der Formel (O)CN-$A_3$-NC(O), wobei $A_3$ ausgewählt ist aus der Gruppe bestehend aus:
  - einer linearen oder verzweigten Alkylengruppe umfassend 2 bis 20 Kohlenstoffatome;
  - einer Cycloalkylen-Alkylen-Cycloalkylen-Gruppe umfassend 6 bis 30 Kohlenstoffatome;

- einer Arylen-Alkylen-Arylen-Gruppe umfassend 6 bis 30 Kohlenstoffatome;
- einer Cycloalkylengruppe umfassend 3 bis 10 Kohlenstoffatome;
- einer Alkylen-Cycloalkylen-Gruppe umfassend 3 bis 15 Kohlenstoffatome; und
- einer Arylengruppe umfassend 6 bis 10 Kohlenstoffatome;

bei einer Temperatur von 40 °C bis 100 °C, vorzugsweise bis 60 °C, in einem Lösemittel.

**11.** Verfahren gemäß Anspruch 9 oder 10, **gekennzeichnet dadurch, dass** das Lösemittel ausgewählt ist aus den Lösemitteln, die das Auflösen der Verbindung gemäß einem der Ansprüche 1 bis 4 ermöglichen, insbesondere DMF oder DMSO, um ein quervernetztes Polyurethan zu erhalten.

**12.** Verfahren gemäß Anspruch 9 oder 10, **gekennzeichnet dadurch, dass** das Lösemittel ausgewählt ist aus den Lösemitteln, die das Auflösen der Verbindung gemäß einem der Ansprüche 1 bis 4 nicht ermöglichen, insbesondere THF, um ein lineares Polyurethan zu erhalten.

**13.** Verwendung der Polymere gemäß einem der Ansprüche 5 bis 8 für die Vektorisierung, Einkapselung oder molekulare Wiedererkennung, insbesondere im medizinischen, pharmazeutischen oder kosmetischen Bereich; die chromatographische Auftrennung, insbesondere im analytischen Bereich; oder für die Herstellung von Adhesiven, von Cotensiden oder von Beschichtungen.

**14.** Intermediat entsprechend der folgenden Formel (I'):

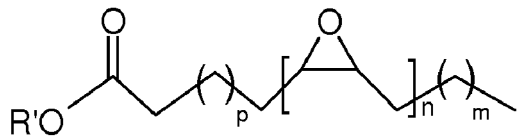

(I')

wobei:

- R' ausgewählt ist aus Zuckern oder Zuckeralkoholen;
- n 1 bis 3 ist;
- m 1 bis 6 ist; und
- p 1 bis 9 ist.

## Claims

**1.** A compound of formula (I):

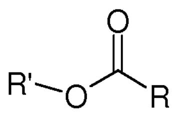

(I)

wherein:

- R represents a linear or branched alkyl group, comprising from 8 to 27 carbon atoms, said alkyl group being substituted with at least two hydroxyl groups, and may optionally contain one unsaturation; and
- R' is selected from sugars or sugar-alcohols, notably from the group consisting of threose, erythrose, deoxyribose, ribose, xylose, ribulose, lyxose, glucose, methyl glucoside, mannose, fructose, idose, sorbose, galactose, allose, maltose, lactose, isomaltose, isomaltulose, cellobiose, saccharose, raffinose, melezitose, sorbitol, isomalt, xylitol, mannitol and arabinitol.

**2.** The compound according to claim 1, wherein R fits the formula (A):

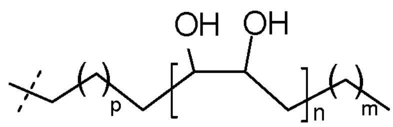

wherein:

- n is comprised from 1 to 3;
- m is comprised from 1 to 6;
- p is comprised from 1 to 9.

**3.** The compound according to any of claims 1 or 2, **characterized in that** R' fits one of the following formulae:

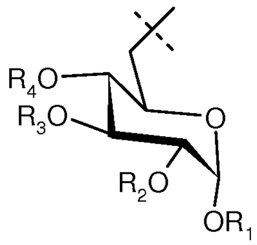
(B)

wherein $R_1$, $R_2$, $R_3$ and $R_4$ represent, independently of each other: H; an alkyl group comprising from 1 to 12 carbon atoms; a group $CH_3C(O)$-; an arylalkyl group comprising from 6 to 12 carbon atoms; a group $((C_1\text{-}C_6)alkyl)_3$-Si- or $R_1$ and $R_2$, $R_2$ and $R_3$ , or $R_3$ and $R_4$, form together an isopropylidene group;
or

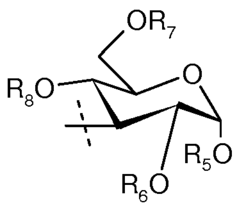
(B')

wherein $R_5$, $R_6$, $R_7$ and $R_8$ represent, independently of each other: H; an alkyl group comprising from 1 to 12 carbon atoms; a group $CH_3C(O)$-; an arylalkyl group comprising from 6 to 12 carbon atoms; a group $((C_1\text{-}C_6)alkyl)_3$-Si- or $R_5$ and $R_6$ or $R_7$ and $R_8$, form together an isopropylidene group,
or

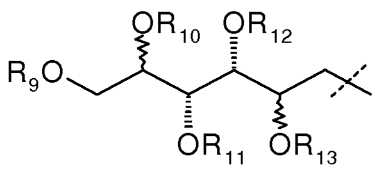
(C)

wherein $R_9$, $R_{10}$, $R_{11}$, $R_{12}$ and $R_{13}$ represent, independently of each other: H; an alkyl group comprising from 1 to 12 carbon atoms; a group $CH_3C(O)$-; an arylalkyl group comprising from 6 to 12 carbon atoms; a group $((C_1\text{-}C_6)alkyl)_3$-Si- or $R_9$ and $R_{10}$, or $R_{10}$ and $R_{11}$, or $R_{11}$ and $R_{12}$, or $R_{12}$ and $R_{13}$, form together un group isopropylidene;
or

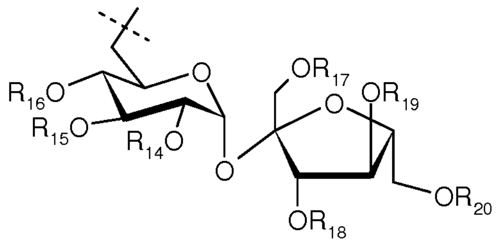

(D)

wherein $R_{14}$, $R_{15}$, $R_{16}$, $R_{17}$, $R_{18}$, $R_{19}$ and $R_{20}$ represent, independently of each other: H; a group alkyl comprising from 1 to 12 carbon atoms; a group $CH_3C(O)$-; an arylalkyl group comprising from 6 to 12 carbon atoms; un group $((C_1\text{-}C_6)alkyl)_3$-Si- or $R_{14}$ and $R_{17}$ or $R_{19}$ and $R_{20}$ form together an isopropylidene group;
or

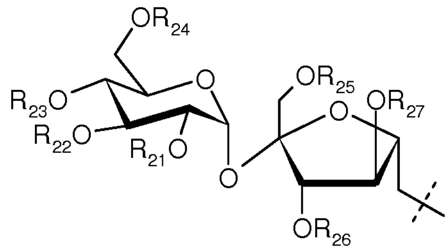

(D')

wherein $R_{21}$, $R_{22}$, $R_{23}$, $R_{24}$, $R_{25}$, $R_{26}$ and $R_{27}$ represent, independently of each other: H; an alkyl group comprising from 1 to 12 carbon atoms; a group $CH_3C(O)$-; an arylalkyl group comprising from 6 to 12 carbon atoms; un group $((C_1\text{-}C_6)alkyl)_3$-Si- or $R_{23}$ and $R_{24}$ or $R_{21}$ and $R_{25}$ or $R_{26}$ and $R_{27}$ form together an isopropylidene group.

4. The compound according to any of claims 1 or 2, **characterized in that** R' fits one of the following formulae:

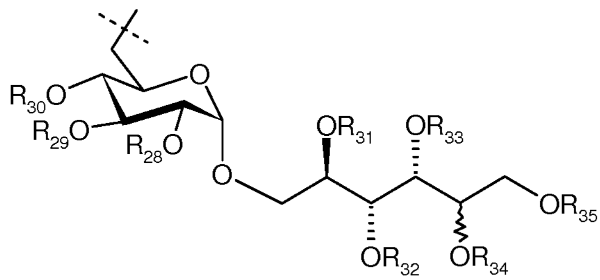

(E)

wherein $R_{28}$, $R_{29}$, $R_{30}$, $R_{31}$, $R_{32}$, $R_{33}$, $R_{34}$ and $R_{35}$ represent, independently of each other: H; an alkyl group comprising from 1 to 12 carbon atoms; a group $CH_3C(O)$-; an arylalkyl group comprising from 6 to 12 carbon atoms; a group $((C_1\text{-}C_6)alkyl)_3$-Si- or $R_{29}$ and $R_{30}$ or $R_{31}$ and $R_{32}$ or $R_{32}$ and $R_{33}$ or $R_{33}$ and $R_{34}$ form together an isopropylidene group;
or

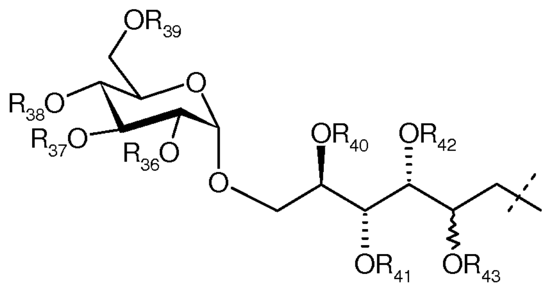

(E')

wherein $R_{36}$, $R_{37}$, $R_{38}$, $R_{39}$, $R_{40}$, $R_{41}$, $R_{42}$ and $R_{43}$ represent, independently of each other: H; an alkyl group comprising from 1 to 12 carbon atoms; a group $CH_3C(O)$-; an arylalkyl group comprising from 6 to 12 carbon atoms; a group $((C_1\text{-}C_6)alkyl)_3$-Si- or $R_{37}$ and $R_{38}$ or $R_{38}$ and $R_{39}$ or $R_{40}$ and $R_{41}$ or $R_{41}$ and $R_{42}$,ou $R_{42}$ and $R_{43}$ form together an isopropylidene group.

**5.** A polymer which may be obtained by polymerisation of a compound as defined according to any of claims 1 to 4 and of a (poly)isocyanate, notably a diisocyanate fitting the formula (O)CN-$A_3$-NC(O), wherein $A_3$ represents:

- a linear or branched alkyl group, comprising from 2 to 20 carbon atoms; or
- a cycloalkyl-alkyl-cycloalkyl group, comprising from 6 to 30 carbon atoms; or
- an aryl-alkyl-aryl group, comprising from 6 to 30 carbon atoms; or
- a cycloalkyl group, comprising from 3 to 10 carbon atoms;
- an alkyl-cycloalkyl group, comprising from 3 to 15 carbon atoms; or
- an aryl group comprising from 6 to 10 carbon atoms.

**6.** A polymer which may be obtained by polymerization of a compound as defined according to any of claims 1 to 4, of a diol and of a (poly)isocyanate, notably a diisocyanate fitting la formule (O)CN-$A_3$-NC(O), wherein $A_3$ represents:

- a linear or branched alkyl group, comprising from 2 to 20 carbon atoms; or
- a cycloalkyl-alkyl-cycloalkyl group, comprising from 6 to 30 carbon atoms; or
- an aryl-alkyl-aryl group, comprising from 6 to 30 carbon atoms; or
- a cycloalkyl group, comprising from 3 to 10 carbon atoms;
- an alkyl-cycloalkyl group, comprising from 3 to 15 carbon atoms; or
- an aryl group comprising from 6 to 10 carbon atoms.

**7.** The polymer according to claim 6, **characterized in that** the diol is selected from alkane-diols, polyalkyl-diols, polyether-diols, polyester-diols and diols of the following formulae:

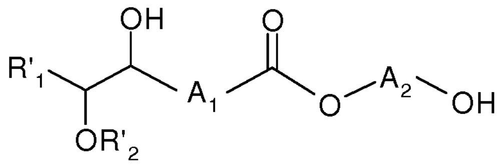

(II)

wherein:

- $R'_1$ represents a linear or branched alkyl group, comprising from 2 to 14 carbon atoms;
- $R'_2$ represents a linear or branched alkyl group, comprising from 1 to 8 carbon atoms;
- $A_1$ represents a linear or branched divalent radical, comprising from 2 to 14 carbon atoms;
- $A_2$ represents a linear or branched divalent radical, comprising from 1 to 10 carbon atoms;

and

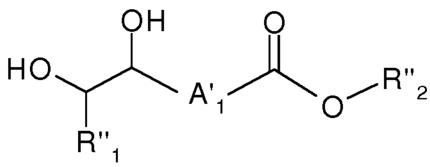

(III)

wherein:

- $R''_1$ represents a linear or branched alkyl group, comprising from 2 to 14 carbon atoms;
- $R''_2$ represents a linear or branched alkyl group, comprising from 1 to 8 carbon atoms;
- $A'_1$ represents a linear or branched divalent radical, comprising from 2 to 14 carbon atoms.

**8.** The polymer according to claim 5, **characterized in that** the polymer fits the following formula:

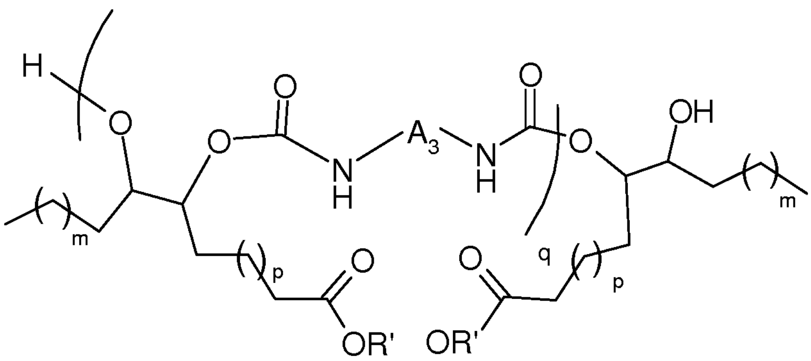

wherein:

- n is 1;
- m is comprised from 1 to 6;
- p is comprised from 1 to 9;
- R' is selected from sugars or sugar-alcohols;
- $A_3$ is selected from the group consisting of:
    - a linear or branched alkyl group, comprising from 2 to 20 carbon atoms;
    - a cycloalkyl-alkyl-cycloalkyl group, comprising from 6 to 30 carbon atoms;
    - an aryl-alkyl-aryl group, comprising from 6 to 30 carbon atoms;
    - a cycloalkyl group, comprising from 3 to 10 carbon atoms;
    - an alkyl-cycloalkyl group, comprising from 3 to 15 carbon atoms; and
    - an aryl group comprising from 6 to 10 carbon atoms;
- q represents an integer comprised between from 2 to 500,000, and preferably from 2 to 50,000, notably from 2 to 5,000, and still preferably from 2 to 50.

**9.** A method for preparing polyurethane, comprising the step for reacting a compound according to any of claims 1 to 4 with a diisocyanate of formula (O)CN-$A_3$-NC(O), according to claim 6, at a temperature comprised from 40°C to 100°C, preferably to 60°C, in a solvent.

**10.** A method for preparing polyurethane, comprising the step for reacting a compound according to any of claims 1 to 4, with:

- a diol;
- a diisocyanate of formula (O)CN-$A_3$-NC(O) wherein $A_3$ is selected from the group consisting of:
    - a linear or branched alkyl group, comprising from 2 to 20 carbon atoms;
    - a cycloalkyl-alkyl-cycloalkyl group, comprising from 6 to 30 carbon atoms;
    - an aryl-alkyl-aryl group, comprising from 6 to 30 carbon atoms;
    - a cycloalkyl group, comprising from 3 to 10 carbon atoms;
    - an alkyl-cycloalkyl group, comprising from 3 to 15 carbon atoms; et
    - an aryl group comprising from 6 to 10 carbon atoms;

at a temperature comprised from 40°C to 100°C, preferably to 60°C, in a solvent.

**11.** The method according to claim 9 or 10, **characterized in that** the solvent is selected from solvents allowing solvation of the compound according to any of claims 1 to 4, notably DMF or DMSO, in order to obtain a network polyurethane.

**12.** The method according to claim 9 or 10, **characterized in that** the solvent is selected from solvents not allowing solvation of the compound according to any of claims 1 to 4, notably THF, in order to obtain a linear polyurethane.

**13.** The use of the polymers according to any of claims 5 to 8, for vectorization, encapsulation or molecular recognition, notably in the medical, pharmaceutical, cosmetic field; chromatographic separation notably in the field of analysis; or for preparing adhesives, co-surfactants or coatings.

**14.** An intermediate compound fitting the following formula (I'):

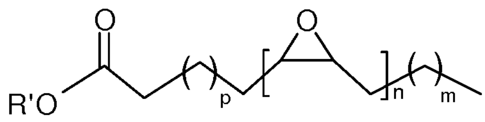

(I')

wherein:

- R' is selected from sugars or sugar-alcohols;
- n is comprised from 1 to 3;
- m is comprised from 1 to 6; and
- p is comprised from 1 to 9.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **NARAIN R. et al.** *Eur. Polym. J.,* 2002, vol. 38, 273-280
- **BERNARD, J. et al.** *Biomacromolecules,* 2005, vol. 7, 232-238
- **BARROS M.T. et al.** *Eur. Polym. J.,* 2009, vol. 45, 295-301
- **NARAIN R. et al.** *Biomacromolecules,* 2003, vol. 4, 1746-1758
- **MUTHUKRISHNAN, H. et al.** *Macromolecules,* 2005, vol. 38, 3108-3119